(19) Europäisches Patentamt
European Patent Office
Office européen des brevets

(11) Veröffentlichungsnummer : **0 412 358 B1**

(12) # EUROPÄISCHE PATENTSCHRIFT

(45) Veröffentlichungstag der Patentschrift :
**29.09.93 Patentblatt 93/39**

(21) Anmeldenummer : **90114303.2**

(22) Anmeldetag : **26.07.90**

(51) Int. Cl.$^5$ : **C07D 249/14,** C07D 401/06, C07D 405/06, C07D 409/06, C07D 413/06, C07D 417/06, A01N 43/653

(54) **3-Amino-5-aminocarbonyl-1,2,4-triazol-Derivate.**

(30) Priorität : **08.08.89 DE 3926119**

(43) Veröffentlichungstag der Anmeldung :
**13.02.91 Patentblatt 91/07**

(45) Bekanntmachung des Hinweises auf die Patenterteilung :
**29.09.93 Patentblatt 93/39**

(84) Benannte Vertragsstaaten :
**BE CH DE FR GB IT LI NL**

(56) Entgegenhaltungen :
**EP-A- 0 048 555**
**EP-A- 0 332 991**
**CHEMICAL ABSTRACTS, Band 85, Nr. 11, 13. September 1976, Columbus, Ohio, USA; VORONKOV M.G. et al.: "Basicity and protonation center of 5(3)-substituted-3(5)-amino-1,2,4-triazole", Seite 487, Spalte 1, Zusammenfassung Nr. 77387u**

(56) Entgegenhaltungen :
**CHEMICAL ABSTRACTS, Band 76, Nr. 11, 13. März 1972, Columbus, Ohio, USA; NICHOLSON S. et al.: "Covalent hydrates as transient species in heterocyclic rearrangements. I. Ring fission of s-triazolopyrazines", Seite 381, Spalte 2, Zusammenfassung Nr. 58494z**

(73) Patentinhaber : **BAYER AG**
**D-51368 Leverkusen (DE)**

(72) Erfinder : **Findeisen, Kurt, Dr.**
**Dünfelder Strasse 28**
**D-5090 Leverkusen 1 (DE)**
Erfinder : **Santel, Hans-Joachim, Dr.**
**Grünstrasse 9a**
**D-5090 Leverkusen 1 (DE)**
Erfinder : **Lürssen, Klaus, Dr.**
**August-Kierspel-Strasse 145**
**D-5060 Bergisch Gladbach 2 (DE)**
Erfinder : **Schmidt, Robert R., Dr.**
**Im Waldwinkel 110**
**D-5060 Bergisch Gladbach 2 (DE)**

EP 0 412 358 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 3-Amino-5-aminocarbonyl-1,2,4-triazol-Derivate, mehrere Verfahren zu ihrer Herstellung und ihre Verwendung als Herbizide.

Es ist bekannt, daß bestimmte Stickstoffheterocyclen (vgl. z.B. K.H. Büchel "Pflanzenschutz und Schädlingsbekämpfung" S. 170, Thieme Verlag Stuttgart 1977) herbizide Eigenschaften besitzen.

Weiter sind bestimmte substituierte Triazole Gegenstand einer eigenen vorgängigen, jedoch nicht vorveröffentlichten Patentanmeldung (vgl. DE-P 38 09 053 vom 18. März 1988).

Die herbizide Wirksamkeit von bekannten Verbindungen ist jedoch nicht in allen Anwendungsgebieten völlig zufriedenstellend.

Es wurden nun neue 3-Amino-5-aminocarbonyl-1,2,4-triazol-Derivate der allgemeinen Formel (I) gefunden,

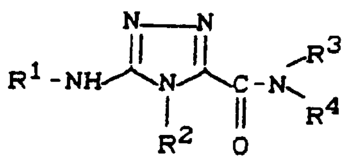

(I)

in welcher

$R^1$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im geradkettigen oder verzweigten Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im geradkettigen oder verzweigten Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/ oder Schwefel steht, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu

6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem $R^3$ und $R^4$ unabhängig voneinander für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen und außerdem $R^3$ und $R^4$ unabhängig voneinander für jeweils gegebenenfalls einfach oder mehrfach, gleich oder verschieden substituiertes Aralkyl, Aroyl oder Aryl mit jeweils 6 bis 10 Kohlenstoffatomen im Arylteil und gegebenenfalls 1 bis 8 Kohlenstoffatomen im geradkettigen oder verzweigten Alkylteil stehen, wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkyl sulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 oder 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen gefunden, wobei

die in der vorgängigen, jedoch nicht vorveröffentlichten Patentanmeldung DE-P 38 09 053 speziell aufgeführten Verbindungen, bei denen

1. $R^1 = CH_3$, $R^2 = CH_3$, $R^3 = H$, $R^4$ = Cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-Phenyl-ethyl;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$;
6. $R^1 = CH_3$, $R^2 = CH_3$, $R^3 = H$, $R^4 = -CH(CH_3)\text{-}CH{=}N\text{-}OCH_3$ und
7. $R^1 = CH_3$, $R^2 = CH_3$, $R^3 = H$,

$R^4$ = 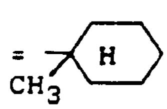

ausgenommen sind.

Weiter wurde gefunden, daß man die neuen Verbindungen der allgemeinen Formel (I) erhält, wenn man

(a) Aminoguanidine der allgemeinen Formel (II)

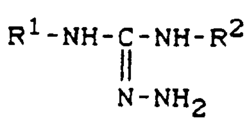 (II)

in welcher

$R^1$ und $R^2$ die oben angegebene Bedeutung haben,

und/oder Tautomere zu den Verbindungen der Formel (II) und/oder Säureaddukte von Verbindungen der Formel (II) oder von deren Tautomeren

mit Oxalsäureesteramiden der allgemeinen Formel (III)

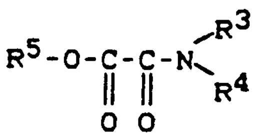

(III)

in welcher

$R^3$ und $R^4$ die oben angegebenen Bedeutungen haben und

$R^5$ für Alkyl steht,

gegebenenfalls in Gegenwart eines Verdünnungsmittels und gegebenenfalls in Gegenwart eines Reaktionshilfsmittels umsetzt, oder wenn man

(b) Chlor-formamidin-Hydrochloride der allgemeinen Formel (IV)

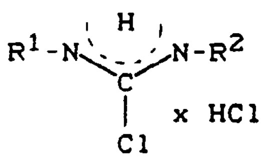

(IV)

in welcher

$R^1$ und $R^2$ die oben angegebenen Bedeutungen haben,

mit Oxalsäure-amid-hydraziden der allgemeien Formel (V)

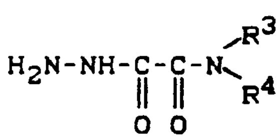

(V)

in welcher

$R^3$ und $R^4$ die oben angegebenen Bedeutungen haben,

gegebenenfalls in Gegenwart eines Verdünnungsmittels und gegebenenfalls in Gegenwart eines Säureakzeptors umsetzt, oder wenn man

(c) Carbodiimide der allgemeinen Formel (VI)

$R^1$-N=C=N-$R^2$ (VI)

in welcher

$R^1$ und $R^2$ die oben angegebenen Bedeutungen haben,

mit Oxalsäure-amid-hydraziden der allgemeinen Formel (V)

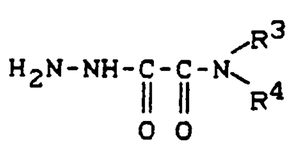

(V)

in welcher

$R^3$ und $R^4$ die oben angegebenen Bedeutungen haben,

gegebenenfalls in Gegenwart eines Verdünnungsmittels und gegebenenfalls in Gegenwart eines Reaktionshilfsmittels umsetzt.

Schließlich wurde gefunden, daß die neuen 3-Amino-5-aminocarbonyl-1,2,4-triazol-Derivate der allgemeinen Formel (I) interessante herbizide Eigenschaften aufweisen.

So zeigen die neuen 3-Amino-5-aminocarbonyl-1,2,4-triazol-Derivate der allgemeinen Formel (I) eine ausgezeichnete Wirkung gegen Problemunkräuter bei guter bis sehr guter Nutzpflanzenverträglichkeit.

Die erfindungsgemäßen 3-Amino-5-aminocarbonyl-1,2,4-triazol-Derivate sind durch die Formel (I) allgemein definiert. Bevorzugt sind Verbindungen der Formel (I), bei welchen

$R^1$ für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, Ethoxymethyl, Ethoxyethyl, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden substituiertes Benzyl, Phenylethyl oder Phenyl steht, wobei als Substituenten infrage kommen; Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ für Methyl, Ethyl, n- oder i-Propyl, n-, i-, s- oder t-Butyl, n- oder i-Pentyl, n- oder i-Hexyl, für Allyl, Propargyl, für Methoxymethyl, Methoxyethyl, Ethoxymethyl, Ethoxyethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio und

$R^3$ und $R^4$ unabhängig voneinander jeweils für Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl oder Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein-bis fünffach, gleich oder verschieden substituiertes Cyclopropyl, Cyclopropylmethyl, Cyclopropylethyl, Cyclobutyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl stehen, wobei als Substituenten jeweils infrage kommen: Fluor, Chlor, Brom, Methyl, Ethyl, n- oder i-Propyl, n-, i-, s-oder t-Butyl, Cyano, Methandiyl, Ethandiyl, Butandiyl oder Butadiendiyl;

$R^3$ und $R^4$ außerdem unabhängig voneinander für im Heterocyclylteil gegebenenfalls ein- bis dreifach, gleich oder verschieden substituiertes Heterocyclylmethyl, Heterocyclylpropyl oder Heterocyclylethyl stehen, wobei als Heterocyclen jeweils infrage kommen:

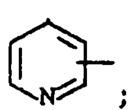 ; 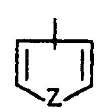 ; 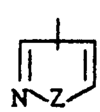 ; 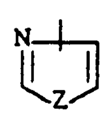 ;

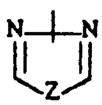 ; 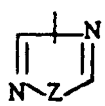 ; 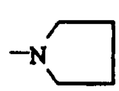 ; 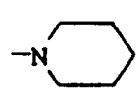 ;

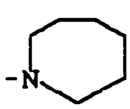 ; 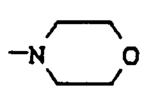 oder 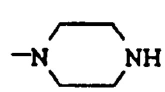 ,

wobei Z jeweils für Sauerstoff oder Schwefel steht und wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;

$R^3$ und $R^4$ außerdem unabhängig voneinander für jeweils gegebenenfalls ein- bis dreifach, gleich oder verschieden substituiertes, gegebenenfalls im Alkylteil geradkettiges oder verzweigtes Benzyl, Phenylethyl, Phenylpropyl, Phenylbutyl, Phenylpentyl, Phenylhexyl, Phenylheptyl Phenylcyanmethyl, Phenylcyanethyl Phenylcyanpropyl, Benzoyl, Phenyl oder Naphthyl stehen, wobei als Phenylsubstituenten jeweils infrage kommen: Fluor, Chlor, Brom, Hydroxy, Cyano, Nitro, Methyl, Ethyl, n- oder i-Propyl, n-, i-, s-oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phen- oxy oder

$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

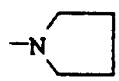 ; 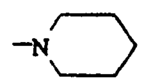 ; 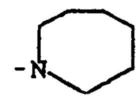 ; 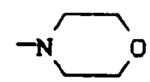 ;

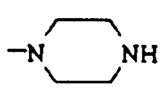 ; 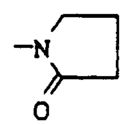 ; 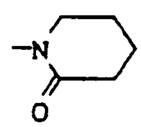 ; 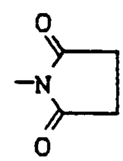 ;

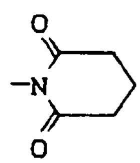 ; 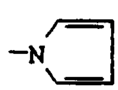 oder 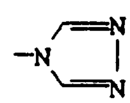 stehen,

wobei als Substituenten jeweils infrage kommen:
Methyl, Ethyl, n- oder i-Propyl, Chlor oder Trifluormethyl,
mit Ausnahme der oben durch Disclaimer ausgenommenen Verbindungen.

Besonders bevorzugt sind Verbindungen der Formel (I), bei welchen

$R^1$ für Methyl, Ethyl, Propyl, Isopropyl oder Cyclohexyl steht,

$R^2$ für Methyl, Ethyl, Propyl, Isopropyl oder Cyclohexyl steht,

$R^3$ für Wasserstoff oder Methyl steht,

$R^4$ für Methyl, Ethyl, n- oder i-Propyl, n-, i-, s- oder t-Butyl, n-, i-, s- oder t-Pentyl, n - oder i-Hexyl, n- oder i-Heptyl, n- oder i-Octyl, n- oder i-Nonyl, n- oder i-Decyl, n- oder i-Dodecyl, 1-Ethyl-propyl, 1,2-Dimethyl-propyl, 1,3-Dimethylbutyl, 1-Methyl-1-ethyl-propyl, 1,1,3,3-Tetramethyl-butyl, 1,2,2-Trimethylpropyl, für Allyl, n- oder i-Butenyl, n- oder i-Pentenyl, n-oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor oder Chlor, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl-bzw. Alkenylteilen oder für jeweils gegebenenfalls ein- bis vierfach, gleich oder verschieden durch Fluor, Chlor, Brom, Methyl, Ethyl, n- oder i-Propyl, n-, i-, s- oder t-Butyl, Cyano, Methandiyl, Ethandiyl, Butandiyl oder Butadiendiyl substituiertes Cyclopropyl, Cyclopropylmethyl, Cyclopropylethyl, Cyclobutyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl steht;

$R^4$ weiterhin für im Heterocyclylteil gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl steht, wobei als Heterocyclen jeweils infrage kommen:

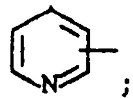 ; 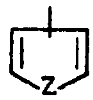 ; 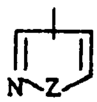 ; 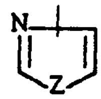 ;

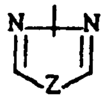 ; 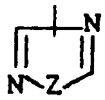 ; 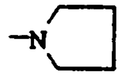 ; 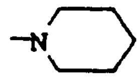 ;

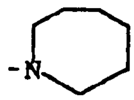 ; 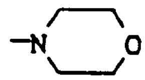 oder 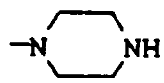 ,

wobei Z jeweils für Sauerstoff oder Schwefel steht,

$R^4$ weiterhin für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifuormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls im Alkylteil geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl, Phenyl oder Naphthyl steht, und $R^4$ außerdem zusammen mit $R^3$ für Tetramethylen oder Pentamethylen stehen kann, mit Ausnahme der oben durch Disclaimer ausgenommenen Verbindungen.

Verwendet man beispielsweise 2-Amino-1-ethyl-3-isopropyl-guanidin und Oxalsäure-methylester-dimethylamid als Ausgangsstoffe, so läßt sich der Reaktionsablauf beim erfindungsgemäßen Verfahren (a) durch das folgende Formelschema darstellen:

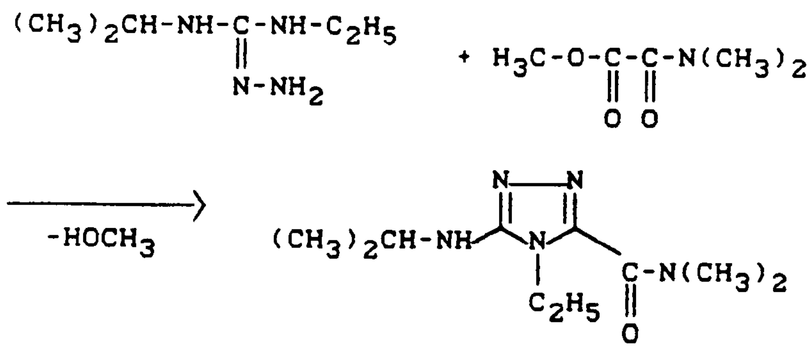


Verwendet man beispielsweise Chlor-dimethylformamidin-Hydrochlorid und Oxalsäure-hydrazid-piperidin als Ausgangsstoffe, so läßt sich der Reaktionsablauf beim erfindungsgemäßen Verfahren (b) durch das folgende Formelschema darstellen:

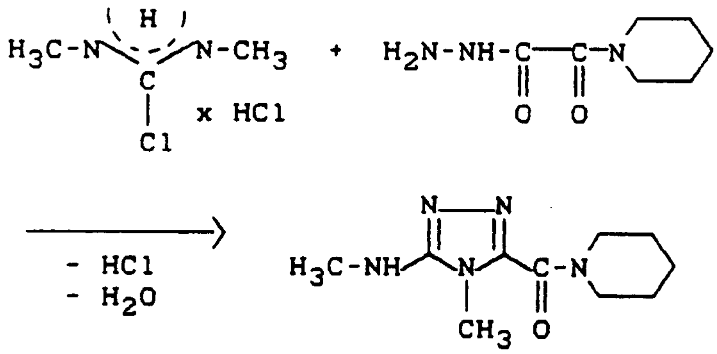


Verwendet man beispielsweise Dicyclohexylcarbodiimid und Oxalsäure-hydrazid-ethylamid als Ausgangsstoffe, so läßt sich der Reaktionsablauf beim erfindungsgemäßen Verfahren (c) durch das folgende Formelschema darstellen:

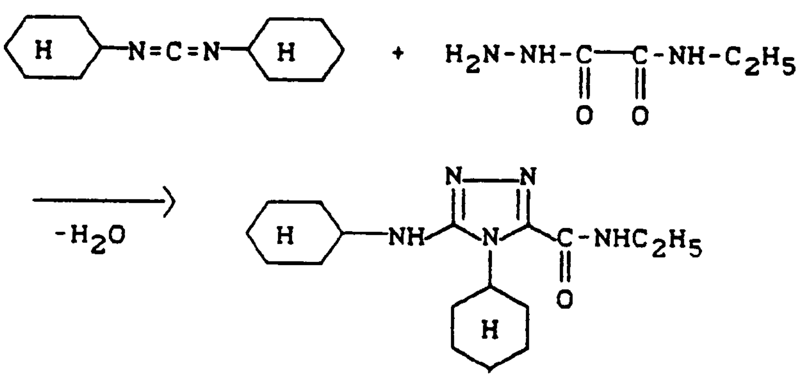


Die beim erfindungsgemäßen Verfahren (a) zur Herstellung von Verbindungen der Formel (I) als Ausgangsstoffe zu verwendenden Aminoguanidine sind durch die Formel (II) allgemein definiert.

In Formel (II) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Bevorzugte Säureaddukte der Verbindungen der Formel (II) sind deren Hydrochloride, Hydrobromide oder Hydroiodide.

Die Ausgangsstoffe der Formel (II) und ihre Säureaddukte sind bekannt und/oder können nach an sich bekannten Verfahren hergestellt werden (vgl. J.Org.Chem. 19 (1954), 1807-1814; Bull. Soc. Chim. France 1975, 1649-1653).

Man erhält die Verbindungen der Formel (II) beispielsweise auch, wenn man 2-Amino-1,1-dimethylguanidin-Derivate der allgemeinen Formel (VII)

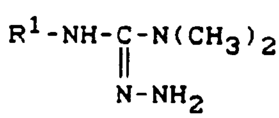 (VII)

in welcher

$R^1$ die oben angegebene Bedeutung hat,

oder Tautomere zu den Verbindungen der Formel (VII) und/oder Säureaddukte (vorzugsweise Hydrochloride, Hydrobromide oder Hydroiodide) von Verbindungen der Formel (VII) oder von deren Tautomeren mit Aminen der allgemeinen Formel (VIII)

$$H_2N\text{-}R^2 \quad (VIII)$$

in welcher

$R^2$ die oben angegebene Bedeutung hat,

in Gegenwart eines Verdünnungsmittels, wie z.B Isopropanol, bei Temperaturen zwischen 20°C und 120°C umsetzt (vgl. die Herstellungsbeispiele).

Die beim erfindungsgemäßen Verfahren (a) weiter als Ausgangsstoffe zu verwendenden Oxalsäureesteramide sind durch die Formel (III) allgemein definiert.

In Formel (III) haben $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^3$ und $R^4$ angegeben wurden und $R^5$ steht vorzugsweise für $C_1$-$C_4$-Alkyl, insbesondere für Methyl oder Ethyl.

Die Ausgangsstoffe der Formel (III) sind bekannt und/oder können nach an sich bekannten Verfahren hergestellt werden (vgl. DE-OS 28 19 878).

Die beim erfindungsgemäßen Verfahren (b) zur Herstellung von Verbindungen der Formel (I) als Ausgangsstoffe zu verwendenden Chlor-formamidin-Hydrochloride sind durch die Formel (IV) allgemein definiert.

In Formel (IV) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Die Ausgangsstoffe der Formel (IV) sind bekannt und/oder können nach an sich bekannten Verfahren hergestellt werden. (vgl. DE-OS 37 09 574, Chem. Ber. 97 (1964), 1232-1245) vgl. auch die Herstellungsbeispiele.

Die bei den erfindungsgemäßen Verfahren (b) und (c) als Ausgangsstoffe zu verwendenden Oxalsäureamid-hydrazide sind durch die Formel (V) allgemein definiert.

In Formel (V) haben $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^3$ und $R^4$ angegeben wurden.

Die Ausgangsstoffe der Formel (V) sind bekannt und/oder können nach an sich bekannten Verfahren hergestellt werden (vgl. EP-A 126 326).

Die beim erfindungsgemäßen Verfahren (c) zur Herstellung von Verbindungen der Formel (I) als Ausgangsstoffe zu verwendenden Carbodiimide sind durch die Formel (VI) allgemein definiert.

In Formel (VI) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Die Ausgangsstoffe der Formel (VI) sind bekannte organische Synthesechemikalien.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Alkohole, wie Methanol, Ethanol oder Propanol.

Das erfindungsgemäße Verfahren (a) wird gegebenenfalls in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 30 °C und 150 °C, vorzugsweise bei Temperaturen zwischen 50 °C und 80 °C.

Zur Durchführung des erfindungsgemäßen Verfahrens (a) setzt man pro Mol an Aminoguanidin der Formel (II) bzw. eines entsprechenden Säureadditionssalzes im allgemeinen 0.8 bis 1.5 Mol, vorzugsweise 0.8 bis 1.2 Mol an Oxalesteramid der Formel (III) und gegebenenfalls 1 bis 5 Mol, vorzugsweise 1 bis 2.5 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Das erfindungsgemäße Verfahren (b) wird gegebenenfalls in Gegenwart eines Verdünnungsmittels durchgeführt. Als Verdünnungsmittel werden vorzugsweise polare organische Lösungsmittel und/oder Wasser eingesetzt. Bevorzugte organische Lösungsmittel sind Alkohole wie Methanol, Ethanol, Propanol, Isopropanol und Butanol, Ether wie Ethylenglycol-dimethylether, Diethylenglycol-dimethylether, Tetrahydrofuran und Dioxan, Etheralkohole wie Ethylenglycol-monomethylether und -monoethylether, Amide wie Formamid und Dimethylformamid, Nitrile wie Acetonitril, Propionitril oder Benzonitril, sowie Pyridin.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z B. Natrium- und Kaliumhydroxid, Erdalkalihydroxide wie z B. Calciumhydroxid, Alkalicarbonate und -alkoholate wie Natrium- und Kaliumcarbonat, Natrium- und Kalium-methylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBU), 1,8-Diazabicyclo-[5,4,0]-undec-7-en (DBO) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 120 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man auf 1 Mol Chlor-formamidin-Hydrochlorid der Formel (IV) im allgemeinen zwischen 0,5 und 1,5 Mol, vorzugsweise zwischen 0,8 und 1,2 Mol, Oxalsäure-amidhydrazid der Formel (V) und zwischen 1 und 5 Moläquivalenten, vorzugsweise zwischen 2 und 3 Moläquivalenten, eines Säureakzeptors ein.

Die Reaktionskomponenten der Formel (II) und (III) werden im allgemeinen bei Raumtemperatur mit dem Verdünnungsmittel vermischt und nach Zugabe eines Säureakzeptors gegebenenfalls bei erhöhter Temperatur - bis zum Ende der Umsetzung gerührt.

Die Aufarbeitung kann nach üblichen Methoden durchgeführt werden (vgl. die Herstellungsbeispiele).

Das erfindungsgemäße Verfahren (c) wird gegebenenfalls in Gegenwart eines Verdünnungsmittels durchgeführt. Es können hierbei die gleichen Verdünnungsmittel verwendet werden, wie sie oben für die erfindungsgemäßen Verfahren (a) und (b) angegeben wurden.

Verfahren (c) wird gegebenenfalls in Gegenwart eines Reaktionshilfsmittels durchgeführt. Es können hierbei die gleichen Reaktionshilfsmittel eingesetzt werden, wie sie oben für das erfindungemäße Verfahren (a) angegeben wurden.

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 20 °C und 120 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, hsparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen wie z.B. in Mais, sowohl im Vorauflauf- als auch im Nachauflauf-Verfahren.

In gewissem Umfang zeigen die Verbindungen der Formel (I) auch fungizide Wirkung, wie z.B. gegen den Apfelschorf (Venturia inaequalis).

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); 3,5-Dibrom-4-hydroxy-benzonitril (BROMOXYNIL); 2-Chlor-4-ethylamino-6-(3-cyanopropylamino)-1,3,5-triazin (CYANAZIN); 4-Amino-6-t-butyl-3-ethylthio-1,2,4-triazin-5(4H)-on (ETHIOZIN); 3,5-Diiod-4-hydroxybenzonitril (IOXYNIL); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); N-(1-Ethylpropyl)-3,4-dimethyl-2,6-dinitroanilin (PENDIMETHALIN); O-(6-Chlor-3-phenylpyridazin-4-yl)-S-octyl-thiocarbonat (PYRIDATE) und 3-[[[[(4-Methoxy-6-methyl-1,3,5-(triazin-2-yl)-amino]- carbonyl]-amino]-sulfonyl]-thiophen-2-carbonsäuremethylester (THIAMETURON). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

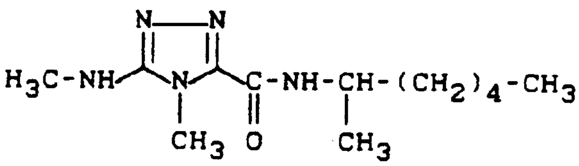

(Verfahren (a))

Eine Mischung aus 24,8 g (0,18 Mol) 2-Amino-1,3-dimethyl-guanidin-Hydrochlorid, 32,9 g (0,15 Mol) Oxalsäureethylester-sec-heptylamid, 16,2 g (0,3 Mol) Natriummethylat und 200 ml Methanol wird 3 Stunden bei Rückflußtemperatur gerührt. Anschließend wird auf Raumtemperatur abgekühlt und filtriert. Das Filtrat wird im Wasserstrahlvakuum eingeengt, der Rückstand in 200 ml Dichlormethan aufgenommen, dreimal mit jeweils 100 ml Wasser gewaschen, über Natriumsulfat getrocknet und filtriert. Vom Filtrat wird das Lösungsmittel im Wasserstrahlvakuum abdestilliert.

Man erhält 24,8 g (65 % der Theorie) 5-Methylamino-4-methyl-4H-1,2,4-triazol-3-yl-carbonsäure-sec-heptylamid vom Schmelzpunkt 101°C-103°C.

Beispiel 2

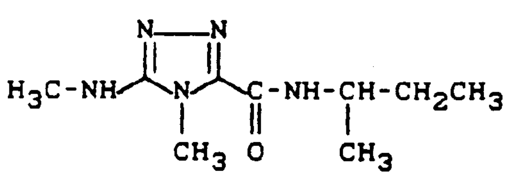

(Verfahren (b))

Eine Mischung aus 7,15 9 (0,05 Mol) Chlor-N,N'-dimethylformamidin-Hydrochlorid, 7,9 g (0,05 Mol) Oxalsäurehydrazid-sec-butylamid, 5,4 g (0,1 Mol) Natriummethylat und 200 ml Butanol wird 60 Minuten bei Rückflußtemperatur gerührt. Anschließend wird auf Raumtemperatur abgekühlt und filtriert. Das Filtrat wird im Wasserstrahlvakuum eingeengt, der Rückstand in 150 ml Dichlormethan aufgenommen, dreimal mit jeweils 100 ml Wasser gewaschen, mit Natriumsulfat getrocknet und filtriert. Vom Filtrat wird das Lösungsmittel im Wasserstrahlvakuum abdestilliert.

Man erhält 4,4 g (40 % der Theorie) 5-Methylamino-4-methyl-4H-1,2,4-triazol-3-yl-carbonsäure-sec-butylamid vom Schmelzpunkt 129°C - 131°C.

Beispiel 3

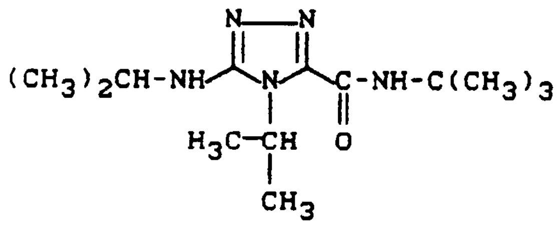

(Verfahren (c))

Eine Mischung aus 12,6 g (0,1 Mol) N,N'-Diisopropylcarbodiimid, 15,9 g (0,1 Mol) Oxalsäure-hydrazid-tert-butylamid, 0,8 g Natriummethylat und 200 ml Butanol wird 3 Stunden bei Rückflußtemperatur gerührt, dann auf Raumtemperatur abgekühlt und filtriert. Das Filtrat wird im Wasserstrahlvakuum eingeengt, der Rückstand in 200 ml Dichlormethan aufgenommen, dreimal mit jeweils 100 ml Wassser gewaschen, mit Natriumsulfat getrocknet und filtriert. Vom Filtrat wird das Lösungsmittel im Wasserstrahlvakuum abdestilliert. Das als Rückstand erhaltene Rohprodukt wird durch Säulenchromatographie (Kieselgel; Cyclohexan/Ethanol 1 : 1) gereinigt und aus Cyclohexan umkristallisiert.

Man erhält 8,7 g (33 % der Theorie) 5-Isopropylamino-4-isopropyl-4H-1,2,4-triazol-3-yl-carbonsäure-tert-butylamid vom Schmelzpunkt 135-137°C.

Analog zu den Beispielen 1 bis 3 und entsprechend der allgemeinen Beschreibung der erfindunsggemäßen Herstellungsverfahren können beispielsweise auch die in der nachstehenden Tabelle 1 aufgeführten Verbindungen der Formel (I) hergestellt werden.

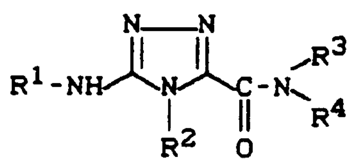

(I)

Tabelle 1: Beispiele für die Verbindungen der Formel (I)

| Bsp.-Nr. | $R^1$ | $R^2$ | $R^3$ | $R^4$ | Schmelzpunkt (°C) |
|---|---|---|---|---|---|
| 4 | $CH_3$ | $CH_3$ | H | $-C(CH_3)_3$ | 166-167 |
| 5 | $CH_3$ | $CH_3$ | H | $-CH(C_2H_5)_2$ | 141-143 |
| 6 | $CH_3$ | $CH_3$ | H | $-CH-CH(CH_3)_2$<br>\|<br>$CH_3$ | 130-132 |
| 7 | $CH_3$ | $CH_3$ | H | $-CH_2CH=CH_2$ | 125-127 |
| 8 | $CH_3$ | $CH_3$ | H | $-CH(CH_3)_2$ | 122-124 |
| 9 | $CH_3$ | $CH_3$ | H | 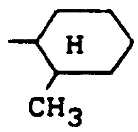 | 132-133 |
| 10 | $CH_3$ | $CH_3$ | H | $-CH_2$ 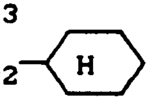 | 146-148 |
| 11 | $CH_3$ | $CH_3$ | H | 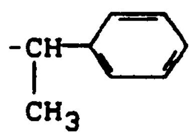 | 118-120 |

Tabelle 1: - Fortsetzung

| Bsp.-Nr. | $R^1$ | $R^2$ | $R^3$ | $R^4$ | Schmelzpunkt (°C) |
|---|---|---|---|---|---|
| 12 | $CH_3$ | $CH_3$ | H | 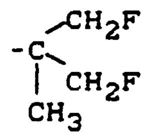 | 159-160 |
| 13 | $CH_3$ | $CH_3$ | H | $-CH_2CH_2Cl$ | 221-223 |
| 14 | $CH_3$ | $CH_3$ | H | 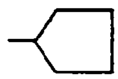 | 160-161 |
| 15 | $CH_3$ | $CH_3$ | H | 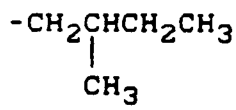 | 79-81 |
| 16 | $CH_3$ | $CH_3$ | H | $-CH_2CH(CH_3)_2$ | 116-118 |
| 17 | $CH_3$ | $CH_3$ | H | 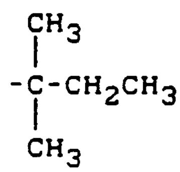 | 150-152 |
| 18 | $CH_3$ | $CH_3$ | H | 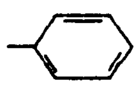 | 219-221 |
| 19 | $CH_3$ | $CH_3$ | H | 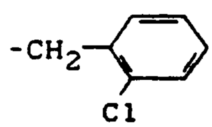 | 150-152 |
| 20 | $CH_3$ | $CH_3$ | H | 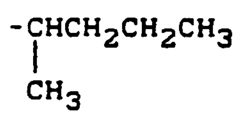 | 118-120 |
| 21 | $CH_3$ | $CH_3$ | H | 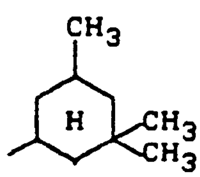 | 152 |

Tabelle 1: - Fortsetzung

| Bsp.-Nr. | $R^1$ | $R^2$ | $R^3$ | $R^4$ | Schmelzpunkt (°C) |
|---|---|---|---|---|---|
| 22 | $CH_3$ | $CH_3$ | H | 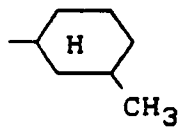 | 137-139 |
| 23 | $CH_3$ | $CH_3$ | H | 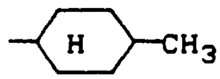 | 132-134 |
| 24 | $CH_3$ | $CH_3$ | H | 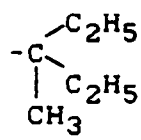 | 115-117 |
| 25 | $CH_3$ | $CH_3$ | H | 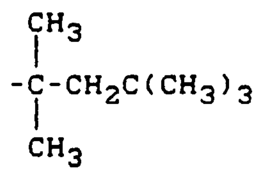 | 97-99 |
| 26 | $CH_3$ | $CH_3$ | H | 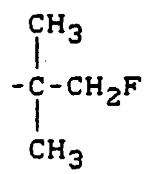 | 124-126 |
| 27 | $CH_3$ | $CH_3$ | H | 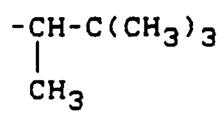 | 90-92 |
| 28 | $CH_3$ | $CH_3$ | H | 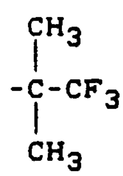 | 191-193 |

Tabelle 1: - Fortsetzung

| Bsp.-Nr. | $R^1$ | $R^2$ | $R^3$ | $R^4$ | Schmelzpunkt (°C) |
|---|---|---|---|---|---|
| 29 | $CH_3$ | $CH_3$ | H | 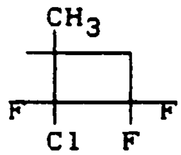 | 109-111 |
| 30 | $CH_3$ | $CH_3$ | H | 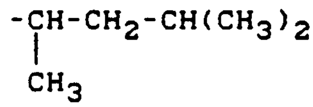 | 114-116 |
| 31 | $CH_3$ | $CH_3$ | H | $-CH_2CH_2CH_2CH_3$ | 118-120 |
| 32 | $CH_3$ | $CH_3$ | H | $-C(CH_3)_3$ | 245-247 |
| 33 | 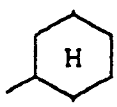 | 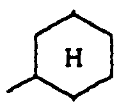 | H | $-C(CH_3)_3$ | 250 |
| 34 | $CH_3$ | $CH_3$ | $—(CH_2)_5—$ | | *) |

*) $^1$H-NMR (DMSO-$D^6$, δ, ppm): 1,50-1,65; 2,80; 6,25.

Ausgangsstoffe der Formel (II):

Beispiel (II-1)

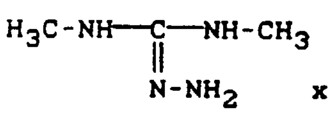 x HCl

152,5 g (1,0 Mol) 2-Amino-1,1,3-trimethyl-guanidin-Hydrochlorid werden in 2,0 Liter Isopropanol auf Rückflußtemperatur erhitzt. Dabei werden innerhalb von 2 Stunden 155 g (5 Mol) Methylamin eingeleitet. Dann wird nach Abkühlen auf 15°C das kristallin angefallene Produkt durch Absaugen isoliert.
Man erhält 98,2 (71 % der Theorie) 2-Amino-1,3-dimethyl-guanidin-Hydrochlorid vom Schmelzpunkt 250°C.
$^1H$-NMR (DMSO-$D^6$, δ, ppm): 2,7-2,8.

Ausgangsstoffe der Formel (IV)

Beispiel (IV-1)

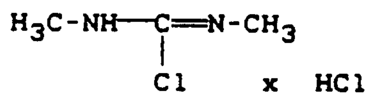

In eine Mischung aus 88 g (1 Mol) N,N'-Dimethylharnstoff und 500 ml Chlorbenzol leitet man bei 80°C innerhalb 1,5 Stunden 110 g (1,1 Mol) Phosgen ein und rührt nach beendetem Einleiten weitere 45 Minuten bis zum Ende der Kohlendioxid-Entwicklung bei 80°C nach. Die Reaktionsmischung wird unter Stickstoff abgekühlt und bei 20°C unter Stickstoff abgesaugt. Das nach dem Einengen des Filtrats erhaltene Festprodukt wird zur Reinigung in Chloroform gelöst und mit Tetrahydrofuran ausgefällt.

Man erhält 34 g (24 % der Theorie) N,N'-Dimethylchloroformamidin-Hydrochlorid vom Schmelzpunkt 156°C-158°C.

Anwendungsbeispiele

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine ausgezeichnete Wirksamkeit in der Bekämpfung von Unkräutern, insbesondere von dikotylen Unkräutern, bei guter Nutzpflanzenselektivität zeigen in diesem Test beispielsweise die Verbindungen gemäß den Herstellungsbeispielen 2, 4, 5, 6, 8, 9, 11, 12, 15, 16, 17, 20, 22, 23 und 34.

Beispiel B

Post-emergence Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich

zur Entwicklung der unbehandelten Kontrolle.

Es bedeuten:

0 % = keine Wirkung (wie unbehandelte Kontrolle)

100 % = totale Vernichtung

Eine ausgezeichnete herbizide Wirkung bei der Bekämpfung von Unkräutern, insbesondere von dikotylen Unkräutern, bei sehr guter Nutzpflanzenselektivität zeigen in diesem Test beispielsweise die Verbindungen gemäß den Herstellungsbeispielen 1, 2, 4, 5, 6, 7, 8, 9, 10, 11, 12, 14, 15, 16, 17, 18, 19, 20, 22, 23, 24 und 34.

## Patentansprüche

1. 3-Amino-5-aminocarbonyl-1,2,4-triazol-Derivate der allgemeinen Formel (I)

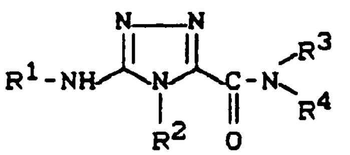

(I)

in welcher

$R^1$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im geradkettigen oder verzweigten Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im geradkettigen oder verzweigten Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/ oder Schwefel steht, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxyiminoalkyl, Alkoxycarbonylalkyl oder Alkoxycarbonylalkenyl mit jeweils bis zu 6 Kohlenstoffatomen in den einzelnen Alkyl bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem $R^3$ und $R^4$ unabhängig voneinander für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/ oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen und außerdem $R^3$ und $R^4$ unabhängig voneinander für jeweils gegebenenfalls einfach oder mehrfach, gleich oder verschieden substituiertes Aralkyl, Aroyl oder Aryl mit jeweils 6 bis 10 Kohlenstoffatomen im Arylteil und gegebenenfalls 1 bis 8 Kohlenstoffatomen im geradkettigen oder verzweigten Alkylteil stehen, wobei als Arylsubstituenten jeweils infrage kommen: Halogen, Cyano,

Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 oder 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo-oder Thionogruppen, wobei die Verbindungen, in denen

1. $R^1 = CH_3$, $R^2 = CH_3$, $R^3 = H$, $R^4$ = Cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-Phenylethyl;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$ und
6. $R^1 = CH_3$, $R^2 = CH_3$, $R^3 = H$, $R^4 = \text{-}(CH(CH_3)\text{-}CH{=}N\text{-}OCH_3$
7. $R^1 = CH_3$, $R^2 = CH_3$, $R^3 = H$,

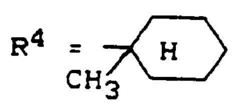

ausgenommen sind.

**2.** 3-Amino-5-aminocarbonyl-1,2,4-triazol-Derivate der Formel (I) gemäß Anspruch 1, bei welchen

$R^1$ für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für

Methoxymethyl, Methoxyethyl, Ethoxymethyl, Ethoxyethyl, für Cyclopropyl, Cyclohexylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden substituiertes Benzyl, Phenylethyl oder Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ für Methyl, Ethyl, n- oder i-Propyl, n-, i-, s- oder t-Butyl, n- oder i-Pentyl, n- oder i-Hexyl, für Allyl, Propargyl, für Methoxymethyl, Methoxyethyl, Ethoxymethyl, Ethoxyethyl, für geradkettiges oder verzweigtes Halogenalkyl mit 1 bis 4 Kohlenstoffatomen und 1 bis 9 gleichen oder verschiedenen Halogenatomen, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio und

$R^3$ und $R^4$ unabhängig voneinander jeweils für Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl oder Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, für jeweils geradkettiges oder verzweigtes Halogenalkenyl oder Halogenalkinyl mit jeweils 3 bis 5 Kohlenstoffatomen und 1 bis 3 Halogenatomen, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl-bzw. Alkenylteilen oder für jeweils gegebenenfalls ein- bis fünffach, gleich oder verschieden substituiertes Cyclopropyl, Cyclopropylmethyl, Cyclopropylethyl, Cyclobutyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl stehen, wobei als Substituenten jeweils infrage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl;

$R^3$ und $R^4$ außerdem unabhängig voneinander für im Heterocyclylteil gegebenenfalls ein- bis dreifach, gleich oder verschieden substituiertes Heterocyclylmethyl, Heterocyclylpropyl oder Heterocyclylethyl stehen, wobei als Heterocyclen jeweils infrage kommen:

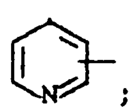 ; 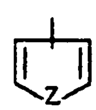 ; 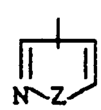 ; 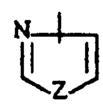 ;

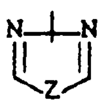 ; 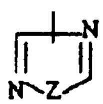 ; 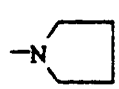 ; 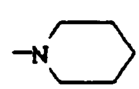 ;

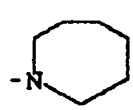 ; 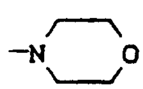 oder 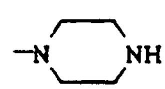 ,

wobei Z jeweils für Sauerstoff oder Schwefel steht und wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;

$R^3$ und $R^4$ außerdem unabhängig voneinander für jeweils gegebenenfalls ein- bis dreifach, gleich oder verschieden substituiertes, gegebenenfalls im Alkylteil geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl,

Phenyl oder Naphthyl stehen, wobei als Phenylsubstituenten jeweils infrage kommen: Fluor, Chlor, Brom, Hydroxy, Cyano, Nitro, Methyl, Ethyl, n- oder i-Propyl, n-, i-, s-oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy oder

$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

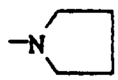 ; 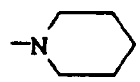 ; 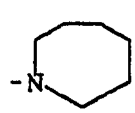 ; 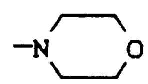 ;

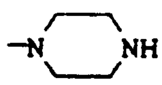 ; 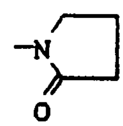 ; 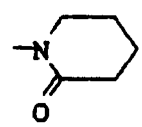 ; 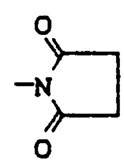 ;

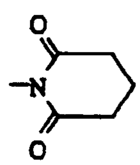 ; 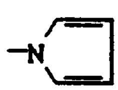 oder 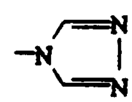 stehen,

wobei als Substituenten jeweils infrage kommen: Methyl, Ethyl, n- oder i-Propyl, Chlor oder Trifluormethyl,

wobei die Verbindungen, in denen

1. $R^1 = CH_3$, $R^2 = CH_3$, $R^3 = H$, $R^4$ = Cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-Phenylethyl;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$;
6. $R^1 = CH_3$, $R^2 = CH_3$, $R^3 = H$, $R^4 = \text{-}(CH(CH_3)\text{-}CH{=}N\text{-}OCH_3$ und
7. $R^1 = CH_3$, $R^2 = CH_3$, $R^3 = H$,

$R^4$ = 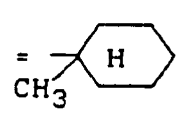

ausgenommen sind.

**3.** 3-Amino-5-aminocarbonyl-1,2,4-triazol-Derivate der Formel (I) gemäß Anspruch 1, in welcher

$R^1$ für Methyl, Ethyl, Propyl, Isopropyl oder Cyclohexyl steht,

$R^2$ für Methyl, Ethyl, Propyl, Isopropyl oder Cyclohexyl steht,

$R^3$ für Wasserstoff oder Methyl steht,

$R^4$ für Methyl, Ethyl, n- oder i-Propyl, n-, i-, s- oder t-Butyl, n-, i-, s- oder t-Pentyl, n- oder i-Hexyl, n- oder i-Heptyl, n- oder i-Octyl, n- oder i-Nonyl, n- oder i-Decyl, n- oder i-Dodecyl, 1-Ethyl-propyl, 1,2-Dimethyl-propyl, 1,3-Dimethyl-butyl, 1-Methyl-1-ethyl-propyl, 1,1,3,3-Trimethyl-butyl, 1,2,2-Trimethyl-propyl, für Allyl, n-oder i-Butenyl, n- oder i-Pentenyl, n-oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen,

insbesondere Fluor oder Chlor, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxyiminoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl-bzw. Alkenylteilen oder für jeweils gegebenenfalls ein-bis vierfach, gleich oder verschieden durch Fluor, Chlor, Brom, Methyl, Ethyl, n- oder i-Propyl, n-, i-, s- oder t-Butyl, Cyano, Methandiyl, Ethandiyl, Butandiyl oder Butadiendiyl substituiertes Cyclopropyl, Cyclopropylmethyl, Cyclopropylethyl, Cyclobutyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl steht;

$R^4$ weiterhin für im Heterocyclylteil gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl steht, wobei als Heterocyclen jeweils infrage kommen:

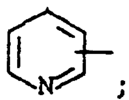 ; 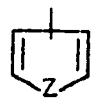 ; 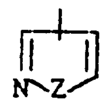 ; 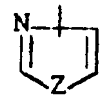 ;

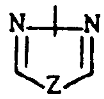 ; 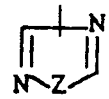 ; 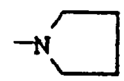 ; 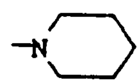 ;

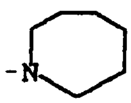 ; 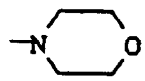 oder 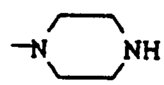 ,

wobei Z jeweils für Sauerstoff oder Schwefel steht,

$R^4$ weiterhin für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls im Alkylteil geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl, Phenyl oder Naphthyl steht, und $R^4$ außerdem zusammen mit $R^3$ für Tetramethylen oder Pentamethylen stehen kann,

wobei die Verbindungen, in denen

1. $R^1 = CH_3$, $R^2 = CH_3$, $R^3 = H$, $R^4$ = Cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-Phenylethyl;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$;
6. $R^1 = CH_3$, $R^2 = CH_3$, $R^3 = H$, $R^4 = \text{-}CH(CH_3)\text{-}CH{=}N\text{-}OCH_3$ und
7. $R^1 = CH_3$, $R^2 = CH_3$, $R^3 = H$,

$R^4$ = 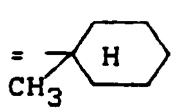

ausgenommen sind.

4. Verfahren zur Herstellung von 3-Amino-5-aminocarbonyl-1,2,4-triazol-Derivaten der allgemeinen Formel (I)

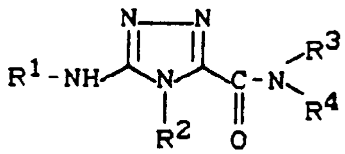

(I)

in welcher

$R^1$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im geradkettigen oder verzweigten Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im geradkettigen oder verzweigten Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/ oder Schwefel steht, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl-bzw. Cycloalkenylteil und gegebenenfalls 1 bis 6 Kohlen stoffatomen im geradkettigen oder verzweigten Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen

oder jeweils zweifach verknüpftes Alkandiyl bzw. Alkendiyl mit jeweils bis zu 4 Kohlenstoffatomen; außerdem $R^3$ und $R^4$ unabhängig voneinander für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel -im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen und außerdem $R^3$ und $R^4$ unabhängig voneinander für jeweils gegebenenfalls einfach oder mehrfach, gleich oder verschieden substituiertes Aralkyl, Aroyl oder Aryl mit jeweils 6 bis 10 Kohlenstoffatomen im Arylteil und gegebenenfalls 1 bis 8 Kohlenstoffatomen im geradkettigen oder versweigten Alkylteil stehen, wobei als Arylsubstituenten jeweils infrage kommen: Halogen, Cyano,

Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkyl sulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 oder 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

wobei die Verbindungen, in denen

1. $R^1 = CH_3$, $R^2 = CH_3$, $R^3 = H$, $R^4$ = Cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-Phenylethyl;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$;
6. $R^1 = CH_3$, $R^2 = CH_3$, $R^3 = H$, $R^4 = \text{-}(CH(CH_3)\text{-}CH{=}N\text{-}OCH_3$ und
7. $R^1 = CH_3$, $R^2 = CH_3$, $R^3 = H$,

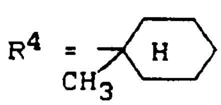

ausgenommen sind,

dadurch gekennzeichnet, daß man

(a) Aminoguanidine der allgemeinen Formel (II)

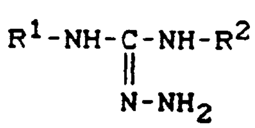

(II)

in welcher

$R^1$ und $R^2$ die oben angegebene Bedeutung haben,

und/oder Tautomere zu den Verbindungen der Formel (II) und/oder Säureaddukte von Verbindungen der Formel (II) oder von deren Tautomeren

mit Oxalsäureesteramiden der allgemeinen Formel (III)

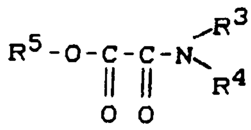

(III)

in welcher

$R^3$ und $R^4$ die oben angegebenen Bedeutungen haben und

$R^5$ für Alkyl steht,

gegebenenfalls in Gegenwart eines Verdünnungsmittels und gegebenenfalls in Gegenwart eines Reaktionshilfsmittels umsetzt, oder daß man

(b) Chlor-formamidin-Hydrochloride der allgemeinen Formel (IV)

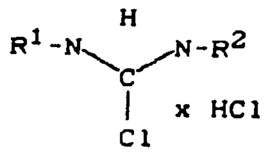

(IV)

in welcher

$R^1$ und $R^2$ die oben angegebenen Bedeutungen haben,

mit Oxalsäure-amid-hydraziden der allgemeinen Formel (V)

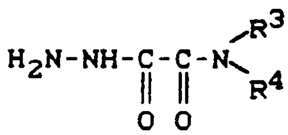

(V)

in welcher

$R^3$ und $R^4$ die oben angegebenen Bedeutungen haben,

gegebenenfalls in Gegenwart eines Verdünnungsmittels und gegebenenfalls in Gegenwart eines Säureakzeptors umsetzt, oder daß man

(c) Carbodiimide der allgemeinen Formel (VI)

$$R^1\text{-}N{=}C{=}N\text{-}R^2 \qquad (VI)$$

in welcher

$R^1$ und $R^2$ die oben angegebenen Bedeutungen haben,

mit Oxalsäure-amid-hydraziden der allgemeinen Formel (V)

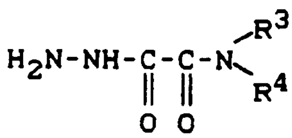

(V)

in welcher

$R^3$ und $R^4$ die oben angegebenen Bedeutungen haben,

gegebenenfalls in Gegenwart eines Verdünnungsmittels und gegebenenfalls in Gegenwart eines Reaktionshilfsmittels umsetzt.

5. Herbizide Mittel, gekennzeichnet durch einen Gehalt an mindestens einem 3-Amino-5-aminocarbonyl-1,2,4-triazol-Derivat der Formel (I) gemäß Anspruch 1 oder 4.

6. Verfahren zur Bekämpfung von unerwünschten Pflanzen, dadurch gekennzeichnet, daß man 3-Amino-5-aminocarbonyl-1,2,4-triazol-Derivate der Formel (I) gemäß Anspruch 1 oder 4 auf die Pflanzen und/oder ihren Lebensraum einwirken läßt.

7. Verwendung von 3-Amino-5-aminocarbonyl-1,2,4-triazol-Derivaten der Formel (I) gemäß Anspruch 1 oder 4 zur Bekämpfung von unerwünschten Pflanzen.

8. Verfahren zur Herstellung von herbiziden Mitteln, dadurch gekennzeichnet, daß man 3-Amino-5-aminocarbonyl-1,2,4-triazol-Derivate der Formel (I) gemäß Anspruch 1 oder 4 mit Streckmitteln und/oder oberflächenaktiven Substanzen vermischt.

## Claims

1. 3-Amino-5-aminocarbonyl-1,2,4-triazole derivatives of the general formula (I)

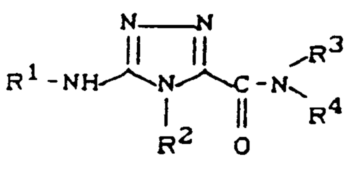

(I)

in which

$R^1$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl having in each case 2 to 8 carbon atoms and 1 to 15, or 13, identical or different halogen atoms, alkoxyalkyl having 1 to 6 carbon atoms in the individual alkyl moieties, or represents cycloalkyl having 3 to 7 carbon atoms, or represents cycloalkylalkyl having 3 to 7 carbon atoms in the cycloalkyl moiety and 1 to 6 carbon atoms in the straight-chain or branched alkyl moiety, or represents aralkyl which has 6 to 10 carbon atoms in the aryl moiety and 1 to 6 carbon atoms in the straight-chain or branched alkyl moiety and which is optionally monosubstituted or polysubstituted by identical or different substituents, or aryl which has 6 to 10 carbon atoms and which is optionally monosubstituted or polysubstituted by identical or different substituents, or heteroaryl which has 2 to 9 carbon atoms and 1 to 3 hetero atoms, in particular nitrogen, oxygen and/or sulphur, and which is optionally monosubstituted or polysubstituted by identical or different substituents, suitable substituents in each case being: halogen, cyano, nitro and in each case straight-chain or branched alkyl, alkoxy, alkylthio, halogenoalkyl, halogenoalkoxy or halogenoalkylthio, in each case having 1 to 4 carbon atoms and where appropriate 1 to 9 identical or different halogen atoms,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in each of the individual alkyl moieties, or represents cycloalkylalkyl or cycloalkyl, in each case having 3 to 7 carbon atoms in the cycloalkyl moiety and where appropriate 1 to 6 carbon atoms in the straight-chain or branched alkyl moiety, or represents aralkyl or aryl, each of which has 6 to 10 carbon atoms in the aryl moiety and where appropriate 1 to 6 carbon atoms in the straight-chain or branched alkyl moiety and each of which is optionally monosubstituted or polysubstituted by identical or different substituents, suitable aryl substituents in each case being: halogen, cyano, nitro and in each case straight-chain or branched alkyl, alkoxy, alkylthio, halogenoalkyl, halogenoalkoxy or halogenoalkylthio, in each case having 1 to 4 carbon atoms and where appropriate 1 to 9 identical or different halogen atoms, and

$R^3$ and $R^4$ independently of one another in each case represent hydrogen, or in each case represent straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, in each case having 2 to 8 carbon atoms and 1 to 15, or 13, identical or different halogen atoms,

cyanoalkyl having 1 to 8 carbon atoms, hydroxyalkyl having 1 to 8 carbon atoms and 1 to 6 hydroxyl groups, or alkoxyalkyl, alkoximinoalkyl, alkoxycarbonylalkyl or alkoxycarbonylalkenyl, in each case having up to 6 carbon atoms in the individual alkyl moieties, or alkenyl moieties, or alkylaminoalkyl or dialkylaminoalkyl, in each case having 1 to 6 carbon atoms in the individual alkyl moieties, or cycloalkyl, cycloalkylalkyl, cycloalkenyl or cycloalkenylalkyl, each of which has 3 to 8 carbon atoms in the cycloalkyl moiety, or cycloalkenyl moiety, and where appropriate 1 to 6 carbon atoms in the straight-chain or branched alkyl moiety and each of which is optionally monosubstituted or polysubstituted by identical or different substituents, suitable substituents in each case being: halogen, cyano and in each case straight-chain or branched alkyl or halogenoalkyl, in each case having 1 to 4 carbon atoms and where appropriate 1 to 9 identical or different halogen atoms, or in each case double-linked alkanediyl, or alkenediyl, in each case having up to 4 carbon atoms; $R^3$ and $R^4$ furthermore independently of one another represent heterocyclylalkyl which has 1 to 6 carbon atoms in the straight-chain or branched alkyl moiety and 1 to 9 carbon atoms as well as 1 to 3 hetero atoms - in particular nitrogen, oxygen and/or sulphur - in the heterocyclyl moiety and each of which is optionally monosubstituted or polysubstituted in the heterocyclyl moiety by identical or different substituents, suitable substituents being: halogen, cyano, nitro, and in each case straight-chain or branched alkyl, alkoxy, alkylthio, halogenoalkyl, halogenoalkoxy, halogenoalkylthio or alkoxycarbonyl, in each case having 1 to 5 carbon atoms and where appropriate 1 to 9 identical or different halogen atoms, and $R^3$ and $R^4$ furthermore independently of one another represent aralkyl, aroyl or aryl, each of which has 6 to 10 carbon atoms in the aryl moiety and where appropriate 1 to 8 carbon atoms in the straight-chain or branched alkyl moiety and each of which is optionally monosubstituted or polysubstituted by identical or different substituents, suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, in each case having 1 to 6 carbon atoms and where appropriate 1 to 9 identical or different halogen atoms, cycloalkyl having 3 to 6 carbon atoms or phenoxy, and suitable alkyl substituents which may be possible being: halogen or cyano, or

$R^3$ and $R^4$ together with the nitrogen atom to which they are bonded represent a five- to ten-membered heterocycle which can optionally contain 1 or 2 further hetero atoms, in particular nitrogen, oxygen and/or sulphur, and which is optionally monosubstituted or polysubstituted by identical or different substituents, suitable substituents being: halogen, and in each case straight-chain or branched alkyl or halogenoalkyl, in each case having 1 to 4 carbon atoms and where appropriate 1 to 9 identical or different halogen atoms as well as 1 to 2 oxo or thiono groups, with the exception of the compounds in which

1. $R^1 = CH_3$, $R^2 = CH_3$, $R^3 = H$, $R^4$ = cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-phenyl-ethyl;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$ and
6. $R^1 = CH_3$, $R^2 = CH_3$, $R^3 = H$, $R^4 = \text{-}CH(CH_3)\text{-}CH{=}N\text{-}OCH_3$
7. $R^1 = CH_3$, $R^2 = CH_3$, $R^3 = H$,

$R^4$ = 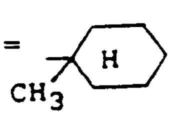

**2.** 3-Amino-5-aminocarbonyl-1,2,4-triazole derivatives of the formula (I) according to Claim 1, in which

$R^1$ represents methyl, ethyl, n- or i-propyl, n-, i-, s- or t-butyl, n- or i-pentyl, allyl, propargyl, or represents in each case straight-chain or branched halogenoalkyl having 1 to 4 carbon atoms, halogenoalkenyl having 3 to 6 carbon atoms or halogenoalkinyl having 3 to 6 carbon atoms and in each case 1 to 9 identical or different halogen atoms, or represents methoxymethyl, methoxyethyl, ethoxymethyl or ethoxyethyl, or represents cyclopropyl, cyclopropylmethyl, cyclopentyl, cyclohexyl, cyclohexylmethyl, cyclohexylethyl or cyclopentylmethyl, or represents benzyl, phenylethyl or phenyl, each of which is optionally monosubstituted to trisubstituted by identical or different substituents, suitable substituents in each case being: fluorine, chlorine, bromine, cyano, nitro, methyl, ethyl, n- or i-propyl, n-, i-, s- or t- butyl, methoxy, ethoxy, methylthio, trifluoromethyl, trifluoromethoxy or trifluoromethylthio,

$R^2$ represents methyl, ethyl, n- or i-propyl, n-, i-, s- or t-butyl, n- or i-pentyl or n- or i-hexyl, or represents allyl or propargyl, or represents methoxymethyl, methoxyethyl, ethoxymethyl or ethoxyethyl, or represents a straight-chain or branched halogenoalkyl having 1 to 4 carbon atoms and 1 to 9 identical or different halogen atoms, or represents cyclopentyl, cyclohexyl, cyclopropyl, cyclopropylmethyl, cyclohexylmethyl or cyclohexylethyl, or represents benzyl or phenyl, each of which is optionally monosubstituted to trisubstituted by identical or different substituents, suitable substituents in each case being: fluorine, chlorine, bromine, cyano, nitro, methyl, ethyl, n- or i- propyl, n-, i-, s- or t-butyl, methoxy, ethoxy, methylthio, trifluoromethyl, trifluoromethoxy or trifluoromethylthio, and

$R^3$ and $R^4$ independently of one another in each case represent hydrogen, methyl, ethyl, n- or i-propyl, n-, i-, s- or t-butyl, in each case straight-chain or branched pentyl, hexyl, heptyl, octyl, nonyl, decyl or dodecyl, or represent allyl, n- or i- butenyl, n- or i-pentenyl, n- or i-hexenyl, propargyl, n- or i-butinyl, n- or i-pentinyl or n- or i- hexinyl, or represent straight-chain or branched halogenoalkyl having 1 to 6 carbon atoms and 1 to 9 identical or different halogen atoms, or represent in each case straight-chain or branched halogenoalkenyl or halogenoalkinyl, in each case having 3 to 5 carbon atoms and 1 to 3 halogen atoms, or represent in each case straight-chain or branched cyanoalkyl having 1 to 6 carbon atoms in the alkyl moiety, hydroxyalkyl having 1 to 6 carbon atoms and 1 to 3 hydroxyl groups, alkoxyalkyl, alkoximinoalkyl, alkoxycarbonylalkyl or alkoxycarbonylalkenyl, alkylaminoalkyl or dialkylaminoalkyl, in each case having up to 4 carbon atoms in the individual alkyl moieties, or alkenyl moieties, or represent cyclopropyl, cyclopropylmethyl, cyclopropylethyl, cyclobutyl, cyclopentyl, cyclohexyl, cycloheptyl, cyclooctyl, cyclohexylmethyl, cyclohexylethyl, cyclohexenyl, cyclohexenylmethyl or cyclohexenylethyl, each of which is optionally monosubstituted to pentasubstituted by identical or different substituents, suitable substituents in each case being: fluorine, chlorine, bromine, methyl, ethyl, n- or i-propyl, n-, i-, s- or t-butyl, cyano, methanediyl, ethanediyl, butanediyl or butadienediyl;

$R^3$ and $R^4$ furthermore independently of one another represent heterocyclylmethyl, heterocyclylpropyl or heterocyclylethyl, each of which is optionally monosub-stituted to trisubstituted in the heterocyclyl moiety by identical or different substituents, suitable heterocycles in each case being:

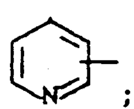 ; 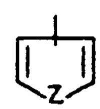 ; 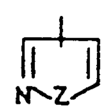 ; 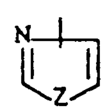 ;

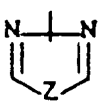 ; 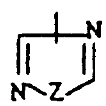 ; 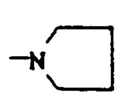 ; 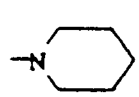 ;

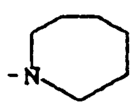 ; 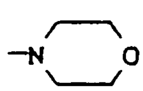 or 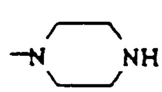 ,

Z in each case representing oxygen or sulphur and suitable substituents in each case being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio;

$R^3$ and $R^4$ furthermore independently of one another represent benzyl, phenylethyl, phenylpropyl, phenylbutyl, phenylpentyl, phenylhexyl, phenylheptyl, phenylcyanomethyl, phenylcyanoethyl, phenylcyanopropyl, benzoyl, phenyl or naphthyl, each of which is straight-chain or branched in the alkyl moiety (where appropriate), and each of which is

optionally monosubstituted to trisubstituted by identical or different substituents, suitable phenyl substituents in each case being: fluorine, chlorine, bromine, hydroxyl, cyano, nitro, methyl, ethyl, n- or i- propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy, or

$R^3$ and $R^4$ together with the nitrogen atom to which they are bonded represent a heterocycle of the formula

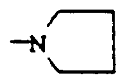 ; 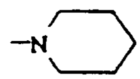 ; 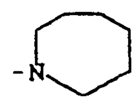 ; 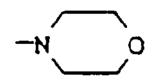 ;

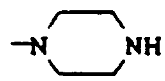 ; 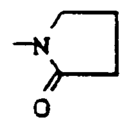 ; 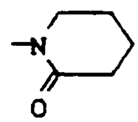 ; 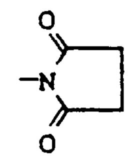 ;

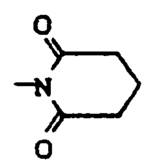 ; 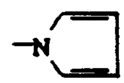 or 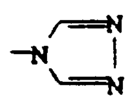 ,

each of which is optionally monosubstituted to trisubstituted by identical or different substituents, suitable substituents in each case being: methyl, ethyl, n- or i-propyl, chlorine or trifluoromethyl,
with the exception of the compounds in which

1. $R^1 = CH_3$, $R^2 = CH_3$, $R^3 = H$, $R^4$ = cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-phenyl-ethly;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$;
6. $R^1 = CH_3$, $R^2 = CH_3$, $R^3 = H$, $R^4 = \text{-}CH(CH_3)\text{-}CH{=}N\text{-}OCH_3$ and
7. $R^1 = CH_3$, $R^2 = CH_3$, $R^3 = H$,

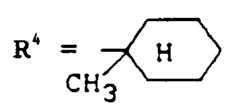

**3.** 3-Amino-5-aminocarbonyl-1,2,4-triazole derivatives of the formula (I) according to Claim 1, in which

$R^1$ represents methyl, ethyl, propyl, isopropyl or cyclohexyl,

$R^2$ represents methyl, ethyl, propyl, isopropyl or cyclohexyl,

$R^3$ represents hydrogen or methyl,

$R^4$ represents methyl, ethyl, n- or i-propyl, n-, i-, s- or t-butyl, n-, i-, s- or t-pentyl, n- or i-hexyl, n- or i-heptyl, n- or i-octyl, n- or i-nonyl, n- or i-decyl, n- or i-dodecyl, 1-ethyl-propyl, 1,2-dimethyl-propyl, 1,3-dimethyl-butyl, 1-methyl-1-ethyl-propyl, 1,1,3,3-tetramethyl-butyl or 1,2,2-trimethyl-propyl, or represents allyl, n- or i- butenyl, n- or i-pentenyl, n- or i-hexenyl, propargyl, n- or i-butinyl, n- or i-pentinyl or n- or i-hexinyl, or represents straight-chain or branched halogenoalkyl having 1 to 6 carbon atoms and 1 to 9 identical or different halogen atoms, in particular fluorine or chlorine, or represents in each case straight-chain or branched halogenoalkenyl or halogenoalkinyl, in each case having 3 to 5 carbon atoms and 1 to 3 halogen atoms, in particular fluorine or chlorine, or represents in each case straight-chain or branched cyanoalkyl having 1 to 6 carbon

atoms in the alkyl moiety, hydroxyalkyl having 1 to 6 carbon atoms and 1 to 3 hydroxyl groups, alkoxyalkyl, alkoximinoalkyl, alkoxycarbonylalkyl or alkoxycarbonylalkenyl, alkylaminoalkyl or dialkylaminoalkyl, in each case having up to 4 carbon atoms in the individual alkyl moieties, or alkenyl moieties, or represents cyclopropyl, cyclopropylmethyl, cyclopropylethyl, cyclobutyl, cyclopentyl, cyclohexyl, cycloheptyl, cyclooctyl, cyclohexylmethyl, cyclohexylethyl, cyclohexenyl, cyclohexenylmethyl or cyclohexenylethyl, each of which is optionally monosubstituted to tetra-substituted by identical or different substituents from the series comprising fluorine, chlorine, bromine, methyl, ethyl, n- or i-propyl, n-, i-, s- or t-butyl, cyano, methanediyl, ethanediyl, butanediyl or butadienediyl;

$R^4$ furthermore represents heterocyclylmethyl, heterocyclylpropyl or heterocyclylethyl, each of which is optionally monosubstituted to tri-substituted in the heterocyclyl moiety by identical or different substituents from the series comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio, suitable hetero cycles in each case being:

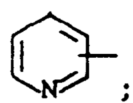 ; 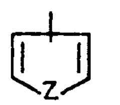 ; 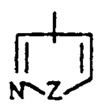 ; 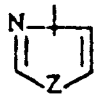 ;

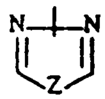 ; 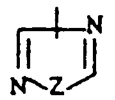 ; 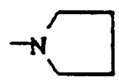 ; 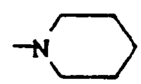 ;

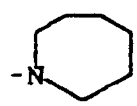 ; 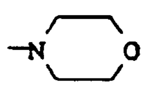 or 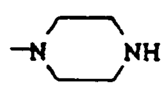 ,

Z in each case representing oxygen or sulphur,

$R^4$ furthermore represents benzyl, phenylethyl, phenylpropyl, phenylbutyl, phenylpentyl, phenylhexyl, phenylheptyl, phenylcyanomethyl, phenylcyanoethyl, phenylcyanopropyl, benzoyl, phenyl or naphthyl, each of which is straight-chain or branched in the alkyl moiety (where appropriate), and each of which is optionally monosubstituted to tri-substituted in the phenyl moiety by identical or different substituents from the series comprising fluorine, chlorine, bromine, hydroxyl, cyano, nitro, methyl, ethyl, n- or i-propyl, n-, i-, s- or t- butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy, and $R^4$ furthermore together with $R^3$ can represent tetramethylene or pentamethylene,

with the exception of the compounds in which

1. $R^1 = CH_3$, $R^2 = CH_3$, $R^3 = H$, $R^4$ = cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-phenyl-ethyl;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$;
6. $R^1 = CH_3$, $R^2 = CH_3$, $R^3 = H$, $R^4 = -CH(CH_3)\ -CH{=}N\text{-}OCH_3$ and
7. $R^1 = CH_3$, $R^2 = CH_3$, $R^3 = H$,

$R^4$ = 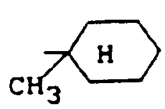

**4.** Process for the preparation of 3-amino-5-amino-carbonyl-1,2,4-triazole derivatives of the general formula (I)

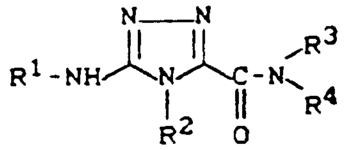

(I)

in which

$R^1$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl having in each case 2 to 8 carbon atoms and 1 to 15, or 13, identical or different halogen atoms, alkoxyalkyl having 1 to 6 carbon atoms in the individual alkyl moieties, or represents cycloalkyl having 3 to 7 carbon atoms, or represents cycloalkylalkyl having 3 to 7 carbon atoms in the cycloalkyl moiety and 1 to 6 carbon atoms in the straight-chain or branched alkyl moiety, or represents aralkyl which has 6 to 10 carbon atoms in the aryl moiety and 1 to 6 carbon atoms in the straight-chain or branched alkyl moiety and which is optionally monosubstituted or polysubstituted by identical or different substituents, or aryl which has 6 to 10 carbon atoms and which is optionally monosubstituted or polysubstituted by identical or different substituents, or heteroaryl which has 2 to 9 carbon atoms and 1 to 3 hetero atoms, in particular nitrogen, oxygen and/or sulphur, and which is optionally monosubstituted or polysubstituted by identical or different substituents, suitable substituents in each case being: halogen, cyano, nitro and in each case straight-chain or branched alkyl, alkoxy, alkylthio, halogenoalkyl, halogenoalkoxy or halogenoalkylthio, in each case having 1 to 4 carbon atoms and where appropriate 1 to 9 identical or different halogen atoms,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in each of the individual alkyl moieties, or represents cycloalkylalkyl or cycloalkyl, in each case having 3 to 7 carbon atoms in the cycloalkyl moiety and where appropriate 1 to 6 carbon atoms in the straight-chain or branched alkyl moiety, or represents aralkyl or aryl, each of which has 6 to 10 carbon atoms in the aryl moiety and where appropriate 1 to 6 carbon atoms in the straight-chain or branched alkyl moiety and each of which is optionally monosubstituted or polysubstituted by identical or different substituents, suitable aryl substituents in each case being: halogen, cyano, nitro and in each case straight-chain or branched alkyl, alkoxy, alkylthio, halogenoalkyl, halogenoalkoxy or halogenoalkylthio, in each case having 1 to 4 carbon atoms and where appropriate 1 to 9 identical or different halogen atoms, and

$R^3$ and $R^4$ independently of one another in each case represent hydrogen, or in each case represent straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, in each case having 2 to 8 carbon atoms and 1 to 15, or 13, identical or different halogen atoms, cyanoalkyl having 1 to 8 carbon atoms, hydroxyalkyl having 1 to 8 carbon atoms and 1 to 6 hydroxyl groups, or alkoxyalkyl, alkoximinoalkyl, alkoxycarbonylalkyl or alkoxycarbonylalkenyl, in each case having up to 6 carbon atoms in the individual alkyl moieties, or alkenyl moieties, or alkylaminoalkyl or dialkylaminoalkyl, in each case having 1 to 6 carbon atoms in the individual alkyl moieties, or cycloalkyl, cycloalkylalkyl, cycloalkenyl or cycloalkenylalkyl, each of which has 3 to 8 carbon atoms in the cycloalkyl moiety, or cycloalkenyl moiety, and where appropriate 1 to 6 carbon atoms in the straight-chain or branched alkyl moiety and each of which is optionally monosubstituted or polysubstituted by identical or different substituents, suitable substituents in each case being: halogen, cyano and in each case straight-chain or branched alkyl or halogenoalkyl, in each case having 1 to 4 carbon atoms and where appropriate 1 to 9 identical or different halogen atoms, or in each case double-linked alkanediyl, or alkenediyl, in each case having up to

4 carbon atoms; $R^3$ and $R^4$ furthermore independently of one another represent heterocyclylalkyl which has 1 to 6 carbon atoms in the straight-chain or branched alkyl moiety and 1 to 9 carbon atoms as well as 1 to 3 hetero atoms - in particular nitrogen, oxygen and/or sulphur - in the heterocyclyl moiety and each of which is optionally monosubstituted or polysubstituted in the heterocyclyl moiety by identical or different substituents, suitable substituents being: halogen, cyano, nitro, and in each case straight-chain or branched alkyl, alkoxy, alkylthio, halogenoalkyl, halogenoalkoxy, halogenoalkylthio or alkoxycarbonyl, in each case having 1 to 5 carbon atoms and where appropriate 1 to 9 identical or different halogen atoms, and $R^3$ and $R^4$ furthermore independently of one another represent aralkyl, aroyl or aryl, each of which has 6 to 10 carbon atoms in the aryl moiety and where appropriate 1 to 8 carbon atoms in the straight-chain or branched alkyl moiety and each of which is optionally monosubstituted or polysubstituted by identical or different substituents, suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, in each case having 1 to 6 carbon atoms and where appropriate 1 to 9 identical or different halogen atoms, cycloalkyl having 3 to 6 carbon atoms or phenoxy, and suitable alkyl substituents which may be possible being: halogen or cyano, or

$R^3$ and $R^4$ together with the nitrogen atom to which they are bonded represent a five- to ten-membered heterocycle which can optionally contain 1 or 2 further hetero atoms, in particular nitrogen, oxygen and/or sulphur, and which is optionally monosubstituted or polysubstituted by identical or different substituents, suitable substituents being: halogen, and in each case straight-chain or branched alkyl or halogenoalkyl, in each case having 1 to 4 carbon atoms and where appropriate 1 to 9 identical or different halogen atoms as well as 1 to 2 oxo or thiono groups,

with the exception of the compounds in which

1. $R^1 = CH_3$, $R^2 = CH_3$, $R^3 = H$, $R^4$ = cyclohexyl;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = 1-phenyl-ethyl;
4. $R^1 = C_2H_5$, $R^2 = CH_3$, $R^3 = H$, $R^4 = C(CH_3)_3$;
5. $R^1 = CH_3$, $R^2 = CH_3$, $R_3 = CH_3$, $R^4 = C(CH_3)_3$;
6. $R^1 = CH_3$, $R^2 = CH_3$, $R^3 = H$, $R^4 = \text{-CH (CH}_3\text{) -CH=N-OCH}_3$ and
7. $R^1 = CH_3$, $R^2 = CH_3$, $R^3 = H$,

$R^4$ = 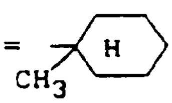

characterized in that

(a) aminoguanidines of the general formula (II)

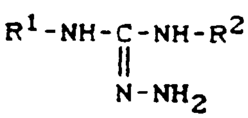

(II)

in which

$R^1$ and $R^2$ have the abovementioned meaning,

and/or tautomers of the compounds of the formula (II) and/or acid adducts of compounds of the formula (II) or of tautomers thereof,

are reacted with oxalamidates of the general formula (III)

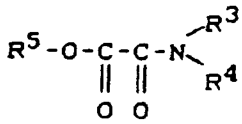

(III)

in which

$R^3$ and $R^4$ have the abovementioned meanings and

$R^5$ represents alkyl,

if appropriate in the presence of a diluent and if appropriate in the presence of a reaction auxiliary, or in that

(b) chloroformamidine hydrochlorides of the general formula (IV)

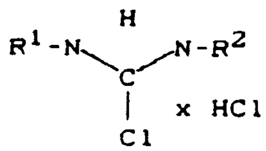

(IV)

in which

$R^1$ and $R^2$ have the abovementioned meanings, are reacted with oxamic acid hydrazides of the general formula (V)

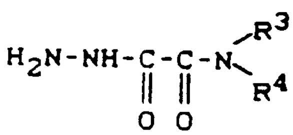

(V)

in which

$R^3$ and $R^4$ have the abovementioned meanings,

if appropriate in the presence of a diluent and if appropriate in the presence of an acid acceptor, or in that

(c) carbodiimides of the general formula (VI)

$$R^1\text{-N=C=N-}R^2 \quad \text{(VI)}$$

in which

$R^1$ and $R^2$ have the abovementioned meanings,

are reacted with oxamic acid hydrazides of the general formula (V)

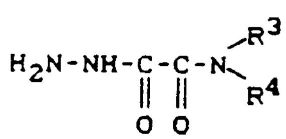

(V)

in which

$R^3$ and $R^4$ have the abovementioned meanings,

if appropriate in the presence of a diluent and if appropriate in the presence of a reaction auxiliary.

5. Herbicidal agents, characterized in that they contain at least one 3-amino-5-aminocarbonyl-1,2,4-triazole derivative of the formula (I) according to Claim 1 or 4.

6. Method of combating undesired plants, characterized in that 3-amino-5-aminocarbonyl-1,2,4-triazole derivatives of the formula (I) according to Claim 1 or 4 are allowed to act on the plants and/or their environment.

7. Use of 3-amino-5-aminocarbonyl-1 ,2 ,4-triazole derivatives of the formula (I) according to Claim 1 or 4 for combating undesired plants.

8. Process for the preparation of herbicidal agents, characterized in that 3-amino-5-aminocarbonyl-1, 2,4-triazole derivatives of the formula (I) according to Claim 1 or 4 are mixed with extenders and/or surface-active substances.

## Revendications

1. Dérivés du 3-amino-5-aminocarbonyl-1,2,4-triazole, répondant à la formule générale (I)

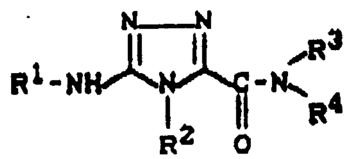

(I)

dans laquelle

$R^1$ représente un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou un groupe halogénalcynyle contenant chacun de 2 à 8 atomes de carbone et de 1 à 15 ou 13 atomes d'halogène identiques ou différents, un groupe alcoxyalkyle contenant de 1 à 6 atomes de carbone dans les fractions alkyle individuelles, chacun de ces groupes étant à chaîne droite ou ramifiée; un groupe cycloalkyle contenant de 3 à 7 atomes de carbone; un groupe cycloalkylalkyle contenant de 3 à 7 atomes de carbone dans la fraction cycloalkyle et de 1 à 6 atomes de carbone dans la fraction alkyle à chaîne droite ou ramifiée; ou encore un groupe aralkyle contenant de 6 à 10 atomes de carbone dans la fraction aryle et de 1 à 6 atomes de carbone dans la fraction alkyle à chaîne droite ou ramifiée, un groupe aryle contenant de 6 à 10 atomes de carbone ou encore un groupe hétéroaryle contenant de 2 à 9 atomes de carbone et de 1 à 3 hétéroatomes, en particulier un atome d'azote, un atome d'oxygène et/ou un atome de soufre, chacun de ces groupes étant éventuellement substitué une ou plusieurs fois de manière identique ou différente, dans lesquels, comme substituants, on envisage respectivement : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée,

$R^2$ représente un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 2 à 8 atomes de carbone et de 1 à 13 atomes d'halogène identiques ou différents, un groupe alcoxyalkyle contenant respectivement de 1 à 6 atomes de carbone dans les fractions alkyle individuelles, chacun de ces groupes étant à chaîne droite ou ramifiée; un groupe cycloalkylalkyle ou un groupe cycloalkyle contenant chacun de 3 à 7 atomes de carbone dans la fraction cycloalkyle et éventuellement de 1 à 6 atomes de carbone dans la fraction alkyle à chaîne droite ou ramifiée; ou encore un groupe aralkyle ou un groupe aryle contenant chacun de 6 à 10 atomes de carbone dans la fraction aryle et éventuellement de 1 à 6 atomes de carbone dans la fraction alkyle à chaîne droite ou ramifiée, chacun de ces groupes étant éventuellement substitué une ou plusieurs fois de manière identique ou différente, dans lesquels, comme substituants du groupe aryle, on envisage respectivement un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée, et

$R^3$ et $R^4$ représentent chacun, indépendamment l'un de l'autre, chacun un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou un groupe halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant de 1 à 8 atomes de carbone et de 1 à 6 groupes hydroxyle, un groupe alcoxyalkyle, un groupe alcoximinoalkyle, un groupe alcoxycarbonylalkyle ou encore un groupe alcoxycarbonylalcényle contenant chacun jusqu'à 6 atomes de carbone dans les fractions alkyle ou alcényle individuelles, un groupe alkylaminoalkyle ou un groupe dialkylaminoalkyle contenant chacun de 1 à 6 atomes de carbone dans les fractions alkyle individuelles, chacun de ces groupes étant à chaîne droite ou ramifiée; ou encore représentent un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou dans la fraction cycloalcényle et éventuellement de 1 à 6 atomes de carbone dans la fraction alkyle à chaîne droite ou ramifiée, chacun de ces groupes étant éventuellement substitué une ou plusieurs fois de manière identique ou différente, dans lesquels, comme substituants, entrent respectivement en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, ou encore un groupe alcanediyle ou un groupe alcènediyle, chacun étant deux fois liés et contenant chacun jusqu'à 4 atomes de carbone, chacun de ces groupes étant à chaîne droite ou ramifiée; en outre, $R^3$ et $R^4$ représentent, indépendamment l'un de l'autre, un groupe hétérocyclylalkyle contenant de 1 à 6 atomes de carbone dans la fraction alkyle à chaîne droite ou ramifiée et de 1 à 9 atomes de carbone, ainsi que de 1 à 3 hétéroatomes - en particulier, un atome d'azote, un atome d'oxygène et/ou un atome de soufre - dans la fraction hétérocyclyle, ce groupe étant éventuellement substitué une ou plusieurs fois de manière identique ou différente dans la fraction hétérocyclyle, dans lesquels, comme substituants, on envisage : un atome d'halogène, un groupe cyano, un groupe nitro; ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; et, en outre, $R^3$ et $R^4$ représentent, indépendamment l'un de l'autre, un groupe aralkyle, un groupe aroyle ou un groupe aryle contenant chacun de 6 à 10 atomes de carbone dans la fraction aryle et éventuellement de 1 à 8 atomes de carbone dans la fraction alkyle à chaîne droite ou ramifiée, chacun de ces groupes étant éventuellement substitué une ou plusieurs fois de manière identique ou différente, dans lesquels, comme substituants du groupe aryle, on envisage, respectivement : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone et éventuellement de 1 à 9 atomes d'halogène identiques ou différents, chacun de ces groupes étant à chaîne droite ou ramifiée; un groupe cycloalkyle contenant de 3 à 6 atomes de carbone ou encore un groupe phénoxy, et dans lesquels, comme substituants du groupe alkyle, on envisage éventuellement : un atome d'halogène ou un groupe cyano, ou bien

$R^3$ et $R^4$ représentent ensemble avec l'atome d'azote auquel ils sont liés, un composé hétérocyclique penta- à décagonal éventuellement substitué une ou plusieurs fois de manière identique ou différente, qui peut contenir éventuellement 1 ou 2 hétéroatomes supplémentaires, en particulier un atome d'azote, un atome d'oxygène et/ou un atome de soufre, dans lequel, comme substituants, on envisage : un atome d'halogène; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; ainsi que de 1 à 2 groupes oxo ou thiono,

les composés dans lesquels

1. $R^1 = CH_3$, $R^2 = CH_3$, $R^3 = H$, $R^4$ = un groupe cyclohexyle;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = un groupe 1-phényléthyle;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$ et
6. $R^1 = CH_3$, $R^2 = CH_3$, $R^3 = H$,
   $R^4 = -CH(CH_3)\text{-}CH{=}N\text{-}OCH_3$
7. $R^1 = CH_3$, $R^2 = CH_3$, $R^3 = H$,

$R^4$ = 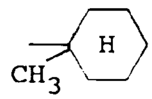

étant exclus.

**2.** Dérivés du 3-amino-5-aminocarbonyl-1,2,4-triazole, répondant à la formule (I) selon la revendication 1, dans lesquels :

$R^1$ représente un groupe méthyle, un groupe éthyle, un groupe n- ou i-propyle, un groupe n-, i-, s- ou t- butyle, un groupe n- ou i-pentyle, un groupe allyle, un groupe propargyle; un groupe halogénalkyle contenant de 1 à 4 atomes de carbone, un groupe halogénalcényle contenant de 3 à 6 atomes de carbone ou un groupe halogénalcynyle contenant de 3 à 6 atomes de carbone et contenant chacun de 1 à 9 atomes d'halogène identiques ou différents, chacun de ces groupes étant à chaîne droite ou ramifiée; un groupe méthoxyméthyle, un groupe méthoxyéthyle, un groupe éthoxyméthyle, un groupe éthoxyéthyle; un groupe cyclopropyle, un groupe éthoxyéthyle; un groupe cyclopropyle, un groupe cyclopropylméthyle, un groupe cyclopentyle, un groupe cyclohexyle, un groupe cyclohexylméthyle, un groupe cyclohexyléthyle, un groupe cyclopentylméthyle; ou encore un groupe benzyle, un groupe phényléthyle ou un groupe phényle, chacun étant éventuellement substitué de 1 à 3 fois de manière identique ou différente, dans lesquels, comme substituants, on envisage : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy ou un groupe trifluorométhylthio,

$R^2$ représente un groupe méthyle, un groupe éthyle, un groupe n- ou i-propyle, un groupe n-, i-, s- ou t- butyle, un groupe n- ou i-pentyle, un groupe n- ou i- hexyle; un groupe allyle, un groupe propargyle; un groupe méthoxyméthyle, un groupe méthoxyéthyle, un groupe éthoxyméthyle, un groupe éthoxyéthyle; un groupe halogénalkyle à chaîne droite ou ramifiée contenant de 1 à 4 atomes de carbone et de 1 à 9 atomes d'halogène identiques ou différents; un groupe cyclopentyle, un groupe cyclohexyle, un groupe cyclopropyle, un groupe cyclopropylméthyle, un groupe cyclohexylméthyle, un groupe cyclohexyléthyle; ou encore un groupe benzyle ou un groupe phényle, chacun étant éventuellement substitué de 1 à 3 fois de manière identique ou différente, dans lesquels, comme substituants, on envisage respectivement : un atome de fluor, un atome de chlore, un atome de brome, un groupe cyano, un groupe nitro, un groupe méthyle, un groupe éthyle, un groupe n- ou i-propyle, un groupe n-, i-, s- ou t- butyle, un groupe méthoxy, un groupe éthoxy, un groupe méthylthio, un groupe trifluorométhyle, un groupe trifluorométhoxy ou un groupe trifluorométhylthio, et

$R^3$ et $R^4$ représentent chacun, indépendamment l'un de l'autre, un atome d'hydrogène, un groupe méthyle, un groupe éthyle, un groupe n- ou i-propyle, un groupe n-, i-, s- ou t-butyle; un groupe pentyle, un groupe hexyle, un groupe heptyle, un groupe octyle, un groupe nonyle, un groupe décyle ou un groupe dodécyle, chacun de ces groupes étant à chaîne droite ou ramifiée; un groupe allyle, un groupe n- ou i-butényle, un groupe n- ou i-pentényle, un groupe n- ou i-hexényle, un groupe propargyle, un groupe n- ou i-butynyle, un groupe n- ou i-pentynyle, un groupe n- ou i-hexynyle, chacun de ces groupes étant à chaîne droite ou ramifiée; un groupe halogénalkyle à chaîne droite ou ramifiée contenant de 1 à 6 atomes de carbone et de 1 à 9 atomes d'halogène identiques ou différents; un groupe halogénalcényle ou un groupe halogénalcynyle contenant chacun de 3 à 5 atomes de carbone et de 1 à 3 atomes d'halogène, chacun de ces groupes étant à chaîne droite ou ramifiée; un groupe cyanalkyle contenant de 1 à 6 atomes de carbone dans la fraction alkyle, un groupe hydroxyalkyle contenant de 1 à 6 atomes de carbone et de 1 à 3 groupes hydroxyle, un groupe alcoxyalkyle, un groupe alcoximinoalkyle, un groupe alcoxycarbonylalkyle ou un groupe alcoxycarbonylalcényle, un groupe alkylaminoalkyle ou un groupe dialkylaminoalkyle contenant chacun jusqu'à 4 atomes de carbone dans les fractions alkyle ou alcényle individuelles, chacun de ces groupes étant à chaîne droite ou ramifiée; ou encore un groupe cyclopropyle, un groupe cyclopropylméthyle, un groupe cyclopropyléthyle, un groupe cyclobutyle, un groupe cyclopentyle, un groupe cyclohexyle, un groupe cycloheptyle, un groupe cyclooctyle, un groupe cyclohexylméthyle, un groupe cyclohexyléthyle, un groupe cyclohexényle, un groupe cyclohexénylméthyle ou un groupe cyclohexényléthyle, chacun de ces groupes étant éventuellement substitué de 1 à 5 fois de manière identique ou différente, dans lesquels, comme substituants, on envisage respectivement : un atome de fluor, un atome de chlore, un atome de brome, un groupe méthyle, un groupe éthyle, un groupe n- ou i-propyle, un groupe n-, i-, s- ou t- butyle, un groupe cyano, un groupe méthanediyle, un groupe éthanediyle, un groupe butanediyle ou un groupe butadiènediyle;

$R^3$ et $R^4$ représentent, en outre, indépendamment l'un de l'autre, un groupe hétérocyclylméthyle, un groupe hétérocyclylpropyle ou un groupe hétérocyclyléthyle éventuellement substitué de 1 à 3 fois de manière identique ou différente dans la fraction hétérocyclyle, dans lesquels, comme composés hétérocycliques, on envisage, respectivement :

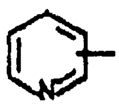 ; 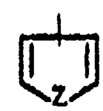 ; 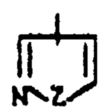 ; 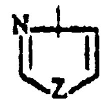 ;

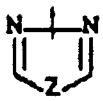 ; 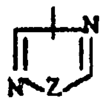 ; 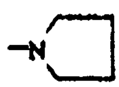 ; 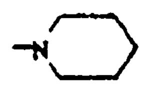 ;

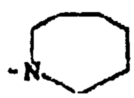 ; 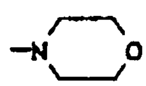 ou 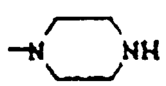 ,

dans lesquels Z représente, respectivement, un atome d'oxygène ou un atome de soufre et dans lesquels, comme substituants, on envisage respectivement un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy ou un groupe trifluorométhylthio;

$R^3$ et $R^4$ représentent, en outre, indépendamment l'un de l'autre, un groupe benzyle, un groupe phényléthyle, un groupe phénylpropyle, un groupe phénylbutyle, un groupe phénylpentyle, un groupe phénylhexyle, un groupe phénylheptyle, un groupe phénylcyanométhyle, un groupe phénylcyanoéthyle, un groupe phénylcyanopropyle, un groupe benzoyle, un groupe phényle ou un groupe naphtyle, chacun de ces groupes étant éventuellement substitué de 1 à 3 fois de manière identique ou différente et étant éventuellement à chaîne droite ou ramifiée dans la fraction alkyle, dans lesquels, comme substituants du groupe phényle, on envisage respectivement : un atome de fluor, un atome de chlore, un atome de brome, un groupe hydroxyle, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy, un groupe trifluorométhylthio, un groupe trifluorométhylsulfinyle, un groupe trifluorométhylsulfonyle, un groupe méthylsulfinyle, un groupe méthylsulfonyle, un groupe acétyle, un groupe propionyle, un groupe méthoxycarbonyle, un groupe

éthoxycarbonyle, un groupe cyclohexyle ou un groupe phénoxy, ou bien

$R^3$ et $R^4$, conjointement avec l'atome d'azote auquel ils sont liés, représentent un composé hétérocyclique éventuellement substitué de 1 à 3 fois de manière identique ou différente, répondant aux formules

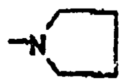 ; 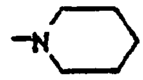 ; 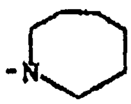 ; 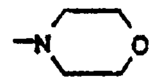 ;

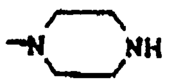 ; 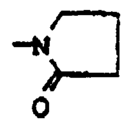 ; 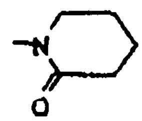 ; 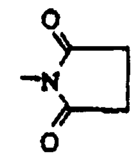 ;

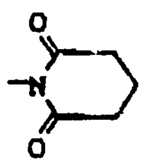 ; 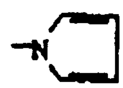 ou 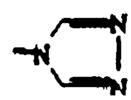

dans lesquelles, comme substituants, on envisage respectivement : un groupe méthyle, un groupe éthyle, un groupe n- ou i-propyle, un atome de chlore ou un groupe trifluorométhyle,

les composés dans lesquels

1. $R^1 = CH_3$, $R^2 = CH_3$, $R^3 = H$, $R^4 =$ un groupe cyclohexyle;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4 =$ un groupe 1-phényléthyle;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$
6. $R^1 = CH_3$, $R^2 = CH_3$, $R^3 = H$, $R^4 = \text{-}CH(CH_3)\text{-}CH=N\text{-}OCH_3$, et
7. $R^1 = CH_3$, $R^2 = CH_3$, $R^3 = H$,

$R^4 =$ 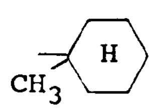

étant exclus.

3. Dérivés du 3-amino-5-aminocarbonyl-1,2,4-triazole, répondant à la formule (I) selon la revendication 1, dans laquelle :

$R^1$ représente un groupe méthyle, un groupe éthyle, un groupe propyle, un groupe isopropyle ou un groupe cyclohexyle,

$R^2$ représente un groupe méthyle, un groupe éthyle, un groupe propyle, un groupe isopropyle ou un groupe cyclohexyle,

$R^3$ représente un atome d'hydrogène ou un groupe méthyle,

$R^4$ représente un groupe méthyle, un groupe éthyle, un groupe n- ou i-propyle, un groupe n-, i-, s- ou t- butyle, un groupe n-, i-, s- ou t-pentyle, un groupe n- ou i-hexyle, un groupe n- ou i-heptyle, un groupe n- ou i-octyle, un groupe n- ou i-nonyle, un groupe n- ou i- décyle, un groupe n- ou i-dodécyle, un groupe 1-éthylpropyle, un groupe 1,2-diméthylpropyle, un groupe 1,3-diméthylbutyle, un groupe l-méthyl-l-éthylpropyle, un groupe 1,1,3,3-tétraméthylbutyle, un groupe 1,2,2-triméthylpropyle; un groupe allyle, un groupe n- ou i- buténylе, un groupe n- ou i-penténylе, un groupe n- ou i-hexényle, un groupe propargyle, un groupe n- ou i- butynyle, un groupe n- ou i-pentynyle, un groupe n- ou i-hexynyle; un groupe halogénalkyle à chaîne droite ou ramifiée contenant de 1 à 6 atomes de carbone et de 1 à 9 atomes d'halogène identiques ou différents, en particulier un atome de fluor ou un atome de chlore; un groupe halogénalcényle ou un groupe halogénalcynyle, chacun étant à chaîne droite ou ramifiée et contenant chacun de 3 à 5 atomes de carbone et de 1 à 3 atomes d'halogène, en particulier un atome de fluor ou un atome de chlore; un groupe cyanalkyle contenant de 1 à 6 atomes de carbone dans la fraction alkyle, un groupe hydroxyalkyle contenant de 1 à 6 atomes de carbone et de 1 à 3 groupes hydroxyle, un groupe alcoxyalkyle, un groupe alcoximinoalkyle, un groupe alcoxycarbonylalkyle ou un groupe alcoxycarbonylalcényle, un groupe alkylaminoalkyle ou un groupe dialkylaminoalkyle contenant chacun jusqu'à 4 atomes de carbone dans les fractions alkyle ou alcényle individuelles, chacun de ces groupes étant à chaîne droite ou ramifiée; ou encore un groupe cyclopropyle, un groupe cyclopropylméthyle, un groupe cyclopropyléthyle, un groupe cyclobutyle, un groupe cyclopentyle, un groupe cyclohexyle, un groupe cycloheptyle, un groupe cyclooctyle, un groupe cyclohexylméthyle, un groupe cyclohexyléthyle, un groupe cyclohexényle, un groupe cyclohexénylméthyle ou un groupe cyclohexényléthyle, chacun de ces groupes étant éventuellement substitué de 1 à 4 fois de manière identique ou différente par un atome de fluor, un atome de chlore, un atome de brome, par un groupe méthyle, un groupe éthyle, un groupe n- ou i-propyle, un groupe n-, i-, s- ou t-butyle, un groupe cyano, un groupe méthanediyle, un groupe éthanediyle, un groupe butanediyle ou un groupe butadiènediyle;

$R^4$ représente, en outre, un groupe hétérocyclylméthyle, un groupe hétérocyclylpropyle ou un groupe hétérocyclyléthyle éventuellement substitué dans la fraction hétérocyclyle de à 3 fois de manière identique ou différente par un atome de fluor, un atome de chlore, un atome de brome, par un groupe cyano, un groupe nitro, un groupe méthyle, un groupe éthyle, un groupe n- ou i-propyle, un groupe n-, i-, s- ou t- butyle, un groupe méthoxy, un groupe éthoxy, un groupe méthylthio, un groupe trifluorométhyle, un groupe trifluorométhoxy ou un groupe trifluorométhylthio, dans lesquels, comme composés hétérocycliques, on envisage, respectivement :

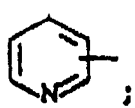 ; 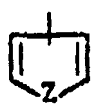 ; 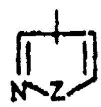 ; 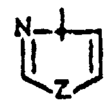 ;

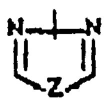 ; 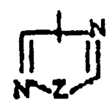 ; 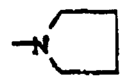 ; 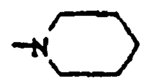 ;

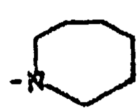 ; 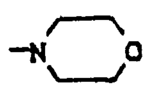 ou 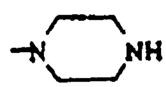 ,

dans lesquels Z représente, respectivement, un atome d'oxygène ou un atome de soufre,

$R^4$ représente, en outre, un groupe benzyle, un groupe phényléthyle, un groupe phénylpropyle, un groupe phénylbutyle, un groupe phénylpentyle, un groupe phénylhexyle, un groupe phénylheptyle, un groupe phénylcyanométhyle, un groupe phénylcyanoéthyle, un groupe phénylcyanopropyle, un groupe benzoyle, un groupe phényle ou un groupe naphtyle, chacun de ces groupes étant éventuellement à chaîne droite ou ramifiée dans la fraction alkyle et chacun de ces groupes étant éventuellement substitué dans la fraction phényle de 1 à 3 fois de manière identique ou différente par un atome de fluor, un atome de chlore, un atome de brome, par un groupe hydroxyle, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy, un groupe trifluorométhylthio, un groupe trifluorométhylsulfinyle, un groupe trifluorométhylsulfonyle, un groupe méthylsulfinyle, un groupe méthylsulfonyle, un groupe acétyle, un groupe propionyle, un groupe méthoxycarbonyle, un groupe éthoxycarbonyle, un groupe cyclohexyle ou un groupe phénoxy; et $R^4$ peut, en outre, représenter, conjointement avec $R^3$, un groupe tétraméthylène ou un groupe pentaméthylène,

les composés dans lesquels

1. $R^1 = CH_3$, $R^2 = CH_3$, $R^3 = H$, $R^4$ = un groupe cyclohexyle;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = un groupe 1-phényléthyle;
4. $R^1 = C_2H_5$, $R^2 = CH_3$, $R^3 = H$, $R^4 = C(CH_3)_3$;
5. $R^1 = CH_3$, $R^2 = CH_3$, $R^3 = CH_3$, $R^4 = C(CH_3)_3$
6. $R^1 = CH_3$, $R^2 = CH_3$, $R^3 = H$,
   $R^4 = \text{-}CH(CH_3)\text{-}CH{=}N{=}OCH_3$ et
7. $R^1 = CH_3$, $R^2 = CH_3$, $R^3 = H$,

$R^4$ = 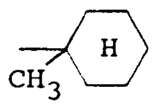

étant exclus.

4. Procédé pour la préparation de dérivés du 3-amino-5-aminocarbonyl-l,2,4-triazole, répondant à la formule générale (I)

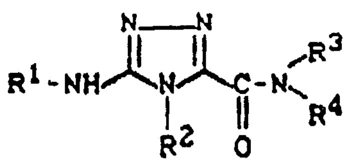

(I)

dans laquelle

$R^1$ représente un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou un groupe halogénalcynyle contenant chacun de 2 à 8 atomes de carbone et de 1 à 15 ou 13 atomes d'halogène identiques ou différents, un groupe alcoxyalkyle contenant de 1 à 6 atomes de carbone dans les fractions alkyle individuelles, chacun de ces groupes étant à chaîne droite ou ramifiée; un groupe cycloalkyle contenant de 3 à 7 atomes de carbone; un groupe cycloalkylalkyle contenant de 3 à 7 atomes de carbone dans la fraction cycloalkyle et de 1 à 6 atomes de carbone dans la fraction alkyle à chaîne droite ou ramifiée; ou encore un groupe aralkyle contenant de 6 à 10 atomes de carbone dans la fraction aryle et de 1 à 6 atomes de carbone dans la fraction alkyle à chaîne droite ou ramifiée, un groupe aryle contenant de 6 à 10 atomes de carbone ou encore un groupe hétéroaryle contenant de 2 à 9 atomes de carbone et de 1 à 3 hétéroatomes, en particulier un atome d'azote, un atome d'oxygène et/ou un atome de soufre, chacun de ces groupes étant éventuellement substitué une ou plusieurs fois de manière identique ou différente, dans lesquels, comme substituants, on envisage respectivement : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée,

$R^2$ représente un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 2 à 8 atomes de carbone et de 1 à 13 atomes d'halogène identiques ou différents, un groupe alcoxyalkyle contenant respectivement de 1 à 6 atomes de carbone dans les fractions alkyle individuelles, chacun de ces groupes étant à chaîne droite ou ramifiée; un groupe cycloalkylalkyle ou un groupe cycloalkyle contenant

chacun de 3 à 7 atomes de carbone dans la fraction cycloalkyle et éventuellement de 1 à 6 atomes de carbone dans la fraction alkyle à chaîne droite ou ramifiée; ou encore un groupe aralkyle ou un groupe aryle contenant chacun de 6 à 10 atomes de carbone dans la fraction aryle et éventuellement de 1 à 6 atomes de carbone dans la fraction alkyle à chaîne droite ou ramifiée, chacun de ces groupes étant éventuellement substitué une ou plusieurs fois de manière identique ou différente, dans lesquels, comme substituants du groupe aryle, on envisage respectivement : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée, et

$R^3$ et $R^4$ représentent chacun, indépendamment l'un de l'autre, un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou un groupe halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant de 1 à 8 atomes de carbone et de 1 à 6 groupes hydroxyle, un groupe alcoxyalkyle, un groupe alcoximinoalkyle, un groupe alcoxycarbonylalkyle ou encore un groupe alcoxycarbonylalcényle contenant chacun jusqu'à 6 atomes de carbone dans les fractions alkyle ou alcényle individuelles, un groupe alkylaminoalkyle ou un groupe dialkylaminoalkyle contenant chacun de 1 à 6 atomes de carbone dans les fractions alkyle individuelles, chacun de ces groupes étant à chaîne droite ou ramifiée; ou encore représentent un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou dans la fraction cycloalcényle et éventuellement de 1 à 6 atomes de carbone dans la fraction alkyle à chaîne droite ou ramifiée, chacun de ces groupes étant éventuellement substitué une ou plusieurs fois de manière identique ou différente, dans lesquels, comme substituants, entrent respectivement en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogéne identiques ou différents, ou encore un groupe alcanediyle ou un groupe alcènediyle, chacun étant deux fois liés et contenant chacun jusqu'à 4 atomes de carbone, chacun de ces groupes étant à chaîne droite ou ramifiée; en outre, $R^3$ et $R^4$ représentent, indépendamment l'un de l'autre, un groupe hétérocyclylalkyle contenant de 1 à 6 atomes de carbone dans la fraction alkyle à chaîne droite ou ramifiée et de 1 à 9 atomes de carbone, ainsi que de 1 à 3 hétéroatomes - en particulier, un atome d'azote, un atome d'oxygène et/ou un atome de soufre - dans la fraction hétérocyclyle, ce groupe étant éventuellement substitué une ou plusieurs fois de manière identique ou différente dans la fraction hétérocyclyle, dans lequel, comme substituants, on envisage : un atome d'halogène, un groupe cyano, un groupe nitro; ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; et, en outre, $R^3$ et $R^4$ représentent, indépendamment l'un de l'autre, un groupe aralkyle, un groupe aroyle ou un groupe aryle contenant chacun de 6 à 10 atomes de carbone dans la fraction aryle et éventuellement de 1 à 8 atomes de carbone dans la fraction alkyle à chaîne droite ou ramifiée, chacun de ces groupes étant éventuellement substitué une ou plusieurs fois de manière identique ou différente, dans lesquels, comme substituants du groupe aryle, on envisage, respectivement un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone et éventuellement de 1 à 9 atomes d'halogène identiques ou différents, chacun de ces groupes étant à chaîne droite ou ramifiée; un groupe cycloalkyle contenant de 3 à 6 atomes de carbone

ou encore un groupe phénoxy, et dans lesquels, comme substituants du groupe alkyle, on envisage éventuellement : un atome d'halogène ou un groupe cyano, ou bien

$R^3$ et $R^4$ représentent ensemble avec l'atome d'azote auquel ils sont liés, un composé hétérocyclique penta- à décagonal éventuellement substitué une ou plusieurs fois de manière identique ou différente, qui peut contenir éventuellement 1 ou 2 hétéroatomes supplémentaires, en particulier un atome d'azote, un atome d'oxygène et/ou un atome de soufre, dans lequel, comme substituants, on envisage : un atome d'halogène; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée, ainsi que de 1 à 2 groupes oxo ou thiono,

les composés dans lesquels

1. $R^1 = CH_3$, $R^2 = CH_3$, $R^3 = H$, $R^4$ = un groupe cyclohexyle;
2. $R^1 = CH_3$, $R^2 = C_2H_5$, $R^3 = H$, $R^4 = CH_2\text{-}C(CH_3)_3$;
3. $R^1 = CH_3$, $R^2 = C_2H_5$, $R^3 = H$, $R^4$ = un groupe 1-phényléthyle;
4. $R^1 = C_2H_5$, $R^2 = CH_3$, $R^3 = H$, $R^4$ , $C(CH_3)_3$;
5. $R^1 = CH_3$, $R^2 = CH_3$, $R^3 = CH_3$, $R^4 = C(CH_3)_3$
6. $R^1 = CH_3$, $R^2 = CH_3$, $R^3 = H$,
   $R^4 = -CH(CH_3)-CH=N-OCH_3$ et
7. $R^1 = CH_3$, $R^2 = CH_3$, $R^3 = H$,

$R^4$ = 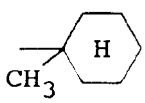

étant exclus,

caractérisé en ce qu'on fait réagir

(a) des aminoguanidines répondant à la formule générale (II)

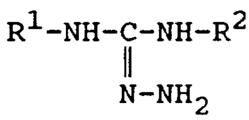

(II)

dans laquelle

$R^1$ et $R^2$ ont la signification indiquée ci-dessus,

et/ou des tautomères par rapport aux composés de formule (II) et/ou des adduits d'acides des composés de formule (II) ou de leurs tautomères

avec des esteramides de l'acide oxalique, répondant à la formule générale (III)

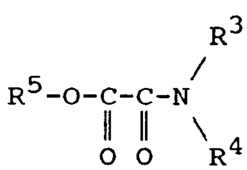

(III)

dans laquelle

$R^3$ et $R^4$ ont les significations indiquées ci-dessus et

$R^5$ représente un groupe alkyle,

éventuellement en présence d'un diluant et éventuellement en présence d'un adjuvant réactionnel, ou bien en ce qu'on fait réagir

(b) des chlorhydrates de chloroformamidine répondant à la formule générale (IV)

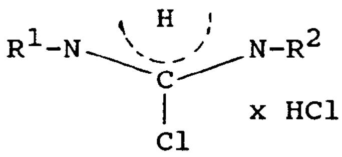

(IV)

dans laquelle

$R^1$ et $R^2$ ont les significations indiquées ci-dessus,

avec des amidohydrazides de l'acide oxalique, répondant à la formule générale (V)

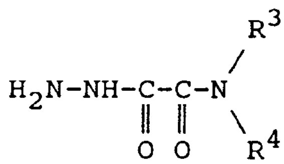

(V)

dans laquelle

$R^3$ et $R^4$ ont les significations indiquées ci-dessus,

éventuellement en présence d'un diluant et éventuellement en présence d'un accepteur d'acides, ou bien en ce qu'on fait réagir

(c) des carbodiimides répondant à la formule générale (VI)

$$R^1\text{-N=C=N-}R^2 \qquad \text{(VI)}$$

dans laquelle

$R^1$ et $R^2$ ont les significations indiquées ci-dessus,

avec des amidohydrazides de l'acide oxalique répondant à la formule générale (V)

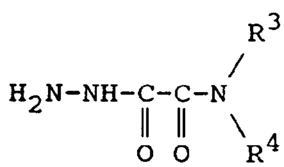

(V)

dans laquelle

$R^3$ et $R^4$ ont les significations indiquées ci-dessus,

éventuellement en présence d'un diluant et éventuellement en présence d'un adjuvant réactionnel.

**5.** Agents herbicides, caractérisés par une teneur en au moins un dérivé du 3-amino-5-aminocarbonyl-1,2,4-triazole, répondant à la formule (I) selon la revendication 1 ou 4.

**6.** Procédé pour lutter contre des plantes non désirées, caractérisé en ce qu'on laisse agir des dérivés du 3-amino-5-aminocarbonyl-1,2,4-triazole de formule (I) selon la revendication 1 ou 4, sur les plantes et/ou sur leur biotope.

**7.** Utilisation de dérivés du 3-amino-5-aminocarbonyl-1,2,4-triazole de formule (I) selon la revendication 1 ou 4, pour lutter contre des plantes non désirées.

**8.** Procédé pour la préparation d'agents herbicides, caractérisé en ce qu'on mélange des dérivés du 3-amino-5-aminocarbonyl-l,2,4-triazole de formule (I) selon la revendication 1 ou 4, avec des diluants et/ou des substances tensioactives.